# FASCICULE DE BREVET EUROPEEN

(11) **EP 2 303 905 B1**
(45) Date de publication et mention de la délivrance du brevet: **28.11.2012**
(21) Numéro de dépôt: 09754068.6
(22) Date de dépôt: 28.05.2009
(51) Int. Cl.: C07H 1/00, C07H 19/06, C07H 19/16, C07H 21/02

(54) **PROCEDE DE SYNTHESE D'ARN PAR VOIE CHIMIQUE**
VERFAHREN ZUR CHEMISCHEN RNA-SYNTHESE
CHEMICAL RNA SYNTHESIS METHOD

(30) Priorité: 29.05.2008 FR 0802928
(43) Date de publication de la demande: 06.04.2011
(73) Titulaire: Centre National de la Recherche Scientifique, 75016 Paris (FR); Université de Montpellier II, 34095 Montpellier Cedex 5 (FR)
(72) Inventeur: DEBART, Françoise, F-34980 Combaillaux (FR); VASSEUR, Jean-Jacques, F-34980 Combaillaux (FR); LAVERGNE, Thomas, F-34980 Saint Clement de Riviere (FR)
(74) Mandataire: Noel, Chantal Odile
(86) Numéro de dépôt international: PCT/FR2009/000624
(87) Numéro de publication internationale: WO 2009/144418

(56) Documents cités:
- CN-A- 1 900 103
- PAREY, NORA ET AL: "First Evaluation of Acyloxymethyl or Acylthiomethyl Groups as Biolabile 2'-O-Protections of RNA" ORGANIC LETTERS , 8(17), 3869-3872 CODEN: ORLEF7; ISSN: 1523-7060, 2006, XP002494287 cité dans la demande
- HOFFMANN, REINHARD W. ET AL: "Synthesis of the trioxadecalin-part of mycalamide B" TETRAHEDRON LETTERS (1993), 34(49), 7903 -6 CODEN: TELEAY; ISSN: 0040-4039, 1993, XP002494288

## Description

L'invention concerne un procédé de synthèse d'ARN, un procédé de libération d'un ARN protégé ainsi qu'un procédé de synthèse de monomères ribonucléotidiques protégés.

Depuis la découverte de l'ARN interférence (ARNi), un besoin crucial de petits ARN synthétiques, en particulier de petits oligoribonucléotides de 21 nucléotides de long (siARN), est apparu pour la recherche biologique et les applications thérapeutiques.

Les unités ribonucléotidiques constituant ces ARN peuvent être des unités ribonucléotidiques naturelles ou modifiées, que ce soit par modification de la nucléobase ou du cycle ribose, comme cela est connu dans l'art, et en particulier comme décrit dans Watts, J. K. et al., Drug Discovery Today, Vol. 13, 19/20, octobre 2008, Schram K. H. et al., Mass Spectrometry Reviews, 1998, 17, 131-251, et Porcher et al., Helvetica Chimica Acta., Vol. 88, 2005, pages 2683-2704.

En comparaison à la synthèse d'ADN, la production d'ARN synthétiques est plus complexe de par la présence de la fonction hydroxyle sur la position 2' du sucre ribose qu'il faut protéger. La recherche du groupe protecteur idéal est un point crucial de la réussite de la synthèse d'ARN. En plus des rendements de couplages plus faibles dans l'assemblage de la chaîne en comparaison à la synthèse d'ADN, la difficulté principale de la chimie de l'ARN résulte de l'instabilité de l'ARN en milieu basique.

C'est pourquoi il est généralement admis dans l'art que la stratégie de synthèse standard utilisée pour la production d'oligodésoxyribonucléotides (petits ADN) où toutes les fonctions réactives de l'ADN sont protégées avec des groupes protecteurs baso-labiles, c'est-à-dire enlevés à la fin du procédé d'élongation chimique par un seul traitement avec une base n'est pas applicable à la production d'oligoribonucléotides (petits ARN).

Comme pour la synthèse d'ADN, les deux voies les plus couramment utilisées à l'heure actuelle pour synthétiser des ARN par voie chimique sont, d'une part, la voie aux phosphoramidites, et, d'autre part, la voie aux hydrogénophosphonates (H-phosphonates).

Dans la voie aux phosphoramidites, on assemble des monomères fonctionnalisés en 3' par un groupe phosphoramidite, l'ARN assemblé ayant alors un lien phosphate internucléotidique 3'-5' protégé, de préférence par un groupe cyanoéthyle.

Le groupe triméthylsilyléthyle (TSE) peut également être utilisé comme groupe protecteur des phosphates, comme enseigné, par exemple dans Parey et al. "First Evaluation of Acyloxymethyl or Acylthiomethyl Groups as Biolabile 2'-O-Protections of RNA", Organic Letters, 2006, Vol.8, No.17, 3869-3872. Mais il est indiqué, dans ce document, que le groupe TSE a été choisi car, contrairement au groupe cyanoéthyle, il n'est pas éliminé dans des conditions basiques mais par des ions fluorures. Il est également indiqué, dans ce document, qu'en réalité le groupe TSE a été éliminé par la solution d'iode utilisée pour l'oxydation effectuée pour obtenir les liens phosphodiesters 3'-5'.

Dans la voie aux hydrogénophosphonates, on assemble des monomères fonctionnalisés en 3' par un groupe hydrogénophosphonate monoester, l'ARN assemblé ayant alors un lien internucléotidique 3'-5' qui est un lien hydrogénophosphonate diester qui est ensuite oxydé en phosphate. Dans cette voie, l'ARN obtenu en fin d'élongation et après oxydation a des liens internucléosidiques 3'-5' phosphates non protégés.

Dans la voie aux phosphoramidites, il est généralement admis dans l'art que la protection de l'hydroxyle en position 2' du sucre ribose ne doit pas être effectuée avec un groupe protecteur baso-labile qui serait enlevé en même temps que le groupe protecteur du phosphate, ce qui entraînerait l'attaque nucléophile par l'hydroxyle en position 2' de l'atome de phosphore dans les liaisons internucléotidiques résultant en une isomérisation 2'-5' des liaisons naturelles 3'-5' ou en une rupture de la liaison 3'-5' dans les conditions de déprotection basique.

Ainsi, T. KEMPE et al, dans "Nucleic Acids Research", 1982, 10, 6695-6714, ont rapporté des rendements de synthèse d'oligoribonucléotides très faibles en protégeant le groupe hydroxyle en position 2' du ribose, avec un groupe acyle tel qu'un groupe acétyle ou benzoyle.

Le groupe *tert*-butyldiméthylsilyle (TBDMS) est certainement le plus utilisé pour la protection de l'hydroxyle en 2' du ribose. Si plusieurs protections ont été proposées pour le remplacer, telles que les groupes triisopropylsilyloxyméthyle (TOM), bis(2-acétoxyéthyloxy)méthyle (ACE), *tert-*butyldithiométhyle (DTM), 1-(2-cyanoéthoxy)éthyle (CEE), 2-cyanoéthoxyméthyle (CEM), 2-(4-toluylsulfonyl)éthoxyméthyle (TEM), lévulinyle et 2-cyanoéthyle, la plupart de ces groupes sont, comme le TBDMS, éliminés par des ions fluorures. Mais une déprotection par des ions fluorures est un obstacle majeur pour obtenir des oligoribonucléotides purs en raison de leur pollution par des sels, menant à des procédures additionnelles longues de purification.

L'invention vise à pallier les inconvénients des procédés de synthèse chimique d'ARN de l'art antérieur, aussi bien de siARN que d'ARN de plus grande longueur, aussi bien que d'ARN comprenant des nucléobases naturelles que des nucléobases modifiées ou que d'ARN comprenant un cycle ribose naturel ou modifié, en proposant un procédé de synthèse d'ARN, qui utilise un groupe protecteur des hydroxyles en position 2' du ribose qui peut être enlevé par une base, sans attaque nucléophile de l'atome de phosphore et sans rupture des liaisons 3'-5' de l'ARN.

Le document CN 1900103A décrit également la synthèse d'ARN et utilise notemment comme groupement protecteur de la fonction OH en 2', un dérivé de type acétoscyméthyle, celui-ci étant cependant hydrolysé en milieu acide.

A cet effet, l'invention propose un procédé de libération d'un ARN simple brin, à partir d'un ARN simple brin protégé et lié par un lien à un support solide, de formule I suivante : 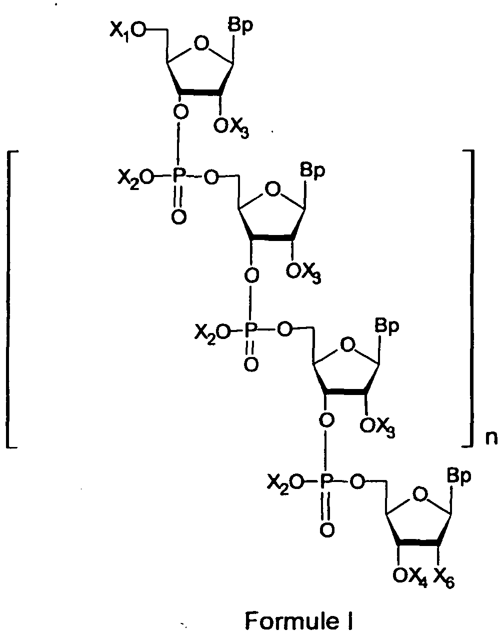 dans laquelle :
- X₁ est H ou un groupe protecteur d'hydroxyle choisi parmi un groupe diméthoxytrityle, un groupe monométhoxytrityle et un groupe pixyle, de préférence un groupe diméthoxytrityle,
- X₂ est H ou un groupe protecteur du phosphate β-éliminable, de préférence un groupe cyanoéthyle,
- X₃ est un groupe baso-labile protecteur des hydroxyles en position 2' du ribose de formule A suivante : 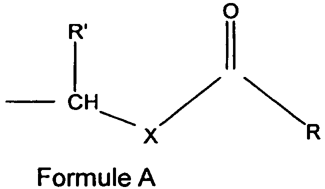 dans laquelle X est O ou S, R' est H ou CH₃, et R est choisi parmi un groupe alkyle en C₁ à C₄ linéaire ou ramifié et un groupe R₁-O-R₂ dans lequel R₁ est un groupe alkyle en C₁ à C₂ et R₂ est un groupe CH₃ ou CH₂CH₂-O-CH₃ ou aryle.
- X₄ représente l'ensemble lien-support solide,
- X₆ est H ou OX₃ ou OAc,
- Bp est une nucléobase thymine naturelle ou modifiée lorsque X₆ est H, uracile naturelle ou modifiée lorsque X₆ est OX₃ ou OAc, adénine naturelle ou modifiée protégée, cytosine naturelle ou modifiée protégée, ou guanine naturelle ou modifiée protégée quelque soit X₆, et
- n est un entier supérieur ou égal à 0,
caractérisé en ce qu'il comprend une étape a) de traitement de l'ARN simple brin protégé lié à un support de formule I avec une base choisie parmi la pipéridine, la 1,8-diazabicyclo[5.4.0] undec-7-ène (DBU), et la triéthylamine, à température ambiante, pour libérer les phosphates des liens internucléotidiques 3'-5' lorsque X₂ est différent de H, suivie d'une étape b) de traitement de l'ARN partiellement libéré obtenu à l'étape a), avec une base choisie parmi l'ammoniaque concentré, la méthylamine, le carbonate de potassium, à température ambiante.

De préférence, dans la formule I, X₃ est un groupe de formule A.

Plus préférablement, dans la formule I, X₃ est choisi parmi un groupe pivaloyloxyméthyle, un groupe isobutyryloxyméthyle, un groupe n-butyryloxyméthyle, un groupe propionyloxyméthyle et un groupe acétyloxyméthyle.

Dans un premier mode de mise en oeuvre préféré du procédé de libération de l'invention, dans la formule I, la nucléobase Bp est l'uracile naturelle ou modifiée.

Dans un second mode de mise en oeuvre préféré du procédé de libération de l'invention, dans la formule I, les quatre nucléobases uracile, adénine, cytosine et guanine, naturelles ou éventuellement et indépendamment l'une de l'autre modifiées sont présentes, et à l'étape b), l'enlèvement du groupe X₃ est effectué préférentiellement par un traitement avec une solution aqueuse d'ammoniaque à 28% puis ajout de 15% en volume, par rapport au volume d'ammoniaque, d'isopropylamine puis évaporation sous pression réduite.

Dans un troisième mode de mise en oeuvre préféré du procédé de libération de l'invention, l'ARN protégé et lié à un support de formule I est lié à un support solide par un lien baso-labile.

Dans un quatrième mode de mise en oeuvre préféré du procédé de libération de l'invention, dans la formule I, les quatre nucléobases naturelles ou éventuellement modifiées, indépendamment l'une de l'autre, thymine, adénine, cytosine, et guanine sont présentes lorsque X₆ est H.

L'invention propose également un procédé de synthèse d'un ARN simple brin caractérisé en ce qu'il comprend les étapes suivantes :
a) liaison, à un support, par un lien, d'un monomère de formule II suivante 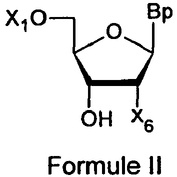 dans laquelle :
   - Bp est une nucléobase naturelle ou modifiée, cette nucléobase étant une nucléobase uracile lorsque X₆ est OX₃ ou OAc, ou une nucléobase thymine lorsque X₆ est H, ou une nucléobase adénine protégée ou une nucléobase cytosine protégée ou une nucléobase guanine protégée, quelque soit X₆
   - X₁ est un groupe diméthoxytrityle,
   - X₆ est H ou un groupe OAc ou OX₃ dans lequel X₃ est un groupe de formule A suivante : 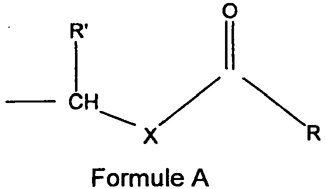
   dans laquelle X est O ou S, R' est H ou CH₃, et R est choisi parmi un groupe alkyle en C₁ à C₄ linéaire ou ramifié et un groupe R₁-O-R₂ dans lequel R₁ est un groupe alkyle en C₁ à C₂ et R₂ est un groupe CH₃ ou CH₂CH₂-O-CH₃ ou aryle,
b) assemblage du monomère de formule II lié à son support obtenu à l'étape a) avec au moins un monomère de formule III suivante : 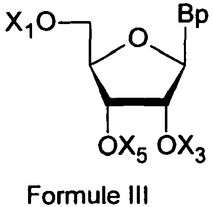 dans laquelle X₁, Bp, et X₃ sont tels que définis pour la formule II et X₅ est un groupe hydrogénophosphonate monoester ou phosphoramidite, de préférence un groupe 2-cyanoéthyl-*N,N*-diisopropylphosphoramidite, ce par quoi on obtient un ARN simple brin protégé lié à un support de formule I,
c) optionnellement, traitement par un milieu acide de l'assemblage obtenu à l'étape b), et
d) libération de l'ARN simple brin protégé lié à un support obtenu à l'étape b) ou à l'étape c), par le procédé de libération de l'invention.

L'invention propose aussi un procédé de synthèse d'un ARN double brin caractérisé en ce qu'il comprend la synthèse d'un ARN simple brin selon le procédé de synthèse d'ARN simple brin de l'invention, et l'hybridation de cet ARN simple brin ainsi synthétisé avec un ARN simple brin ayant une séquence complémentaire.

De préférence, l'ARN double brin est un siARN.

Mais l'invention propose encore un procédé de synthèse d'un monomère de formule III suivante: 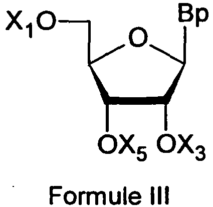 dans laquelle X₁, Bp, X₃ sont tels que définis pour la formule II et X₅ est un groupe hydrogénophosphonate ou phosphoramidite, de préférence un groupe 2-cyanoéthyl-*N,N*-diisopropylphosphoramidite, ce par quoi on obtient un ARN simple brin protégé à un support de formule I,
à partir d'un monomère ribonucléoside de formule IV suivante : 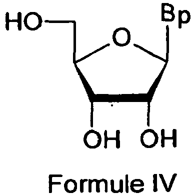 dans laquelle Bp est tel que défini pour la formule II,
caractérisé en ce qu'il consiste en les étapes suivantes :
a) protection des amines exocycliques des nucléobases Bp, lorsque la nucléobase Bp est différente de l'uracile, éventuellement modifiée,
b) protection de l'hydroxyle en position 5' du sucre ribose,
c) protection de l'hydroxyle en position 2' du sucre ribose avec
   un groupe de formule A suivante, et 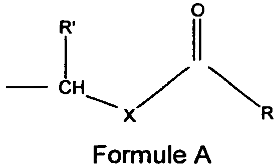 dans laquelle X est O ou S, R' est H ou CH₃, et R est choisi parmi un groupe alkyle en C₁ à C₄ linéaire ou ramifié et un groupe R₁-O-R₂ dans lequel R₁ est un groupe alkyle en C₁ à C₂ et R₂ est un groupe CH₃ ou CH₂CH₂-O-CH₃ ou aryle,
d) fonctionnalisation de l'hydroxyle en position 3' du sucre ribose avec un groupe hydrogénophosphonate monoester ou phosphoramidite, de préférence un groupe 2-cyanoéthyl-*N,N*-diisopropylphosphoramidite.

Plus préférablement, à l'étape c), le groupe de formule A est un groupe pivaloyloxyméthyle ou un groupe *iso*butyryloxyméthyle ou un groupe n-butyryloxyméthyle, ou un groupe propionyloxyméthyle ou un groupe acétyloxyméthyle.

Egalement de préférence, à l'étape b), le groupe protecteur est un groupe diméthoxytrityle.

Dans un premier mode de mise en oeuvre préféré du procédé de synthèse du monomère de formule III selon l'invention, la nucléobase est la cytosine, naturelle ou modifiée, et le groupe protecteur à l'étape a) est un groupe acétyle.

Dans un second mode de mise en oeuvre préféré du procédé de synthèse du monomère de formule III selon l'invention, la nucléobase est l'adénine, naturelle ou modifiée, et le groupe protecteur à l'étape a) est un groupe phénoxyacétyle.

Dans un troisième mode de mise en oeuvre préféré du procédé de synthèse du monomère de formule III selon l'invention, la nucléobase est la guanine, naturelle ou modifiée, et le groupe protecteur à l'étape a) est un groupe *tert*-butylphénoxyacétyle ou *iso*propylphénoxyacétyle.

L'invention sera mieux comprise et d'autres avantages et caractéristiques de celle-ci apparaitront plus clairement à la lecture de la description explicative qui suit.

Dans l'invention, les termes « nucléobase(s) Bp » ou « Bp » désignent une nucléobase uracile, adénine, cytosine ou guanine, éventuellement modifiée, protégée ou non comme cela apparaîtra clairement à l'homme de l'art.

L'invention représente une rupture dans les stratégies antérieures de synthèse d'ARN, quel que soit le nombre d'unités ribonucléotidiques le composant, et que ces unités ribonucléotidiques soient des unités naturelles ou modifiées, par voie chimique, en ce qu'elle utilise des groupements protecteurs des différentes fonctions réactives sur les ribonucléotides, dont en particulier les groupes protecteurs des phosphates des liens internucléotidiques 3'-5, lorsqu'on utilise la voie de synthèse aux phosphoramidites, et les groupes protecteurs des hydroxyles en position 2' des cycles riboses, qui sont enlevés dans des conditions basiques, à la fin de l'élongation de l'ARN. Ceci permet, lorsque les groupements protecteurs des nucléobases, lorsque présents, et le lien liant l'ARN synthétisé protégé au support sont également baso-labiles, d'utiliser une stratégie de synthèse d'ARN entièrement baso-labile. L'invention est donc une rupture du dogme "la synthèse d'ARN est incompatible avec une stratégie entièrement baso-labile".

La caractéristique clé permettant d'utiliser une telle stratégie entièrement baso-labile, est l'utilisation, pour protéger l'hydroxyle en position 2' du sucre ribose, de groupes protecteurs de formule A suivante : 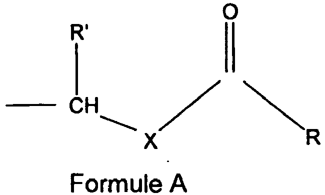 dans laquelle X est O ou S, R' est H ou CH₃, et R est choisi parmi un groupe alkyle en C₁ à C₄ linéaire ou ramifié et un groupe R₁-O-R₂ dans lequel R₁ est un groupe alkyle en C₁ à C₂ et R₂ est un groupe CH₃ ou CH₂CH₂-O-CH₃ ou aryle.

Plus préférablement, ces groupes protecteurs de formule A sont des groupes pivaloyloxyméthyle ou *iso*butyryloxyméthyle ou butyryloxyméthyle ou propionyloxyméthyle ou acétyloxyméthyle.

En effet, l'utilisation de tels groupes protecteurs permet de libérer les hydroxyles en position 2' du ribose de l'ARN voulu par un traitement avec une base, sans rupture de l'ARN et sans contamination par les sels fluorures utilisés dans l'art antérieur.

Les groupements protecteurs pivaloyloxyméthyle, acétyloxyméthyle, pivaloylthiométhyle, et acétylthiométhyle ont déjà été introduits en position 2' du cycle ribose, formant avec l'OH en position 2' du cycle ribose, un groupement *O*-pivaloyloxyméthyle, *O*-acétyloxyméthyle, *O*-pivaloylthiométhyle ou *O*-acétylthiométhyle, respectivement, dans le but d'améliorer certaines propriétés de l'ARN protégé, en particulier sa perméabilité aux membranes cellulaires, sa résistance aux nucléases, comme décrit dans Parey et al, "First Evaluation of Acyloxymethyl or Acylthiomethyl Groups as Biolabile 2'-O-Protections of RNA, Organic Letters", 2006, Vol.8, No.17, 3869-3872.

Dans cet article, les groupes acétalesters, et en particulier les groupes pivaloyloxyméthyle, acétyloxyméthyle, pivaloylthiométhyle et acétylthiométhyle, sont décrits comme des groupes pouvant permettre à l'ARN ainsi fonctionnalisé en position 2' du cycle ribose, de traverser la membrane cellulaire et, là, d'être déprotégé par la cellule elle-même avec son contenu enzymatique. Ces groupements protecteurs étaient donc décrits comme des groupements bio-labiles et rien dans ce document ne suggère que l'utilisation de tels groupements protecteurs permettrait d'utiliser une stratégie de synthèse d'ARN avec protections baso-labiles déprotégé dans des conditions basiques.

De plus, dans ce document, l'introduction sélective du groupement acétalester en position 2' du monomère ribonucléosidique est effectué par une synthèse dans laquelle le réactif de Markiewicz (TIPSiCl₂) bloque simultanément les hydroxyles en positions 5' et 3' du ribose et laisse l'hydroxyle en 2' du ribose libre pour accepter le groupement pivaloyloxyméthyle, acétyloxyméthyle, pivaloylthiométhyle ou acétylthiométhyle, selon le groupement choisi. Cette méthode permet d'éviter l'obtention et la séparation des différents isomères 2' et 3' protégés mais elle requiert au moins sept étapes pour la synthèse de l'ARN protégé.

Bien que ces sept étapes se déroulent avec des rendements élevés, elles sont consommatrices de temps et le TIPSiCl₂ est un réactif coûteux.

L'invention, en contraste, propose un procédé de synthèse du monomère ribonucléosidique dont la nucléobase, et les hydroxyles en position 3' et 5' du ribose sont protégés de manière tout à fait classique dans l'art, l'hydroxyle en position 2' du sucre ribose étant protégé par un groupe acyloxyalkyle ou acylthioalkyle, plus préférablement acyloxyméthyle, qui se déroule en quatre étapes avec un rendement final compris entre 27% et 37%.

Dans ce procédé on part, comme dans l'art antérieur, des composés de formule IV suivante : 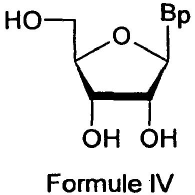 dans laquelle Bp est une nucléobase naturelle ou modifiée, uracile, adénine, cytosine, ou guanine.

La première étape de ce procédé consiste à protéger la nucléobase, lorsque celle-ci est différente de l'uracile.

La seconde étape consiste à protéger l'hydroxyle en position 5' du cycle ribose avec un groupe protecteur classiquement utilisé par l'homme du métier à cet effet. Ces groupes protecteurs classiques utilisés dans l'art pour protéger l'hydroxyle en position 5' du cycle du ribose sont, entre autres, les groupes diméthoxytrityle, monométhoxytrityle et pixyle.

Dans l'invention, le groupe protecteur préféré utilisé est un groupe diméthoxytrityle acido-labile largement utilisé en synthèse d'oligonucléotides.

La troisième étape du procédé de synthèse d'un monomère ribonucléotidique, qui sert d'unité de construction pour l'assemblage d'une chaîne menant à l'obtention d'un ARN simple brin, est la protection de l'hydroxyle en position 2' du ribose par un groupement de formule A suivante : 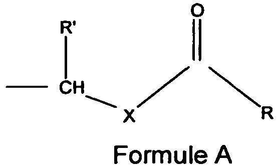 dans laquelle X est O ou S, R' est H ou CH₃, et R est choisi parmi un groupe alkyle en C₁ à C₄ linéaire ou ramifié et un groupe R₁-O-R₂ dans lequel R₁ est un groupe alkyle en C₁ à C₂ et R₂ est un groupe CH₃ ou CH₂CH₂-O-CH₃ ou aryle.

Plus préférablement, le groupe protecteur de l'hydroxyle en position 2' du ribose est un groupe pivaloyloxyméthyle ou un groupe *iso*butyryloxyméthyle, ou un groupe n-butyryloxyméthyle, ou un groupe propionyloxyméthyle, ou un groupe acétyloxyméthyle.

Le plus préférablement, le groupe protecteur de l'hydroxyle en position 2' du ribose est un groupe pivaloyloxyméthyle.

A cette étape, on obtient deux isomères : l'un avec le groupe hydroxyle en position 3' du ribose naturel ou modifié protégé par le groupe de formule A, et l'autre avec le groupe hydroxyle en position 2' du ribose naturel ou modifié protégé par le groupe de formule A.

L'isomère dont l'hydroxyle en position 2' du cycle ribose est protégé, est séparé et la quatrième étape du procédé de synthèse du monomère ribonucléotidique protégé selon l'invention consiste alors à fonctionnaliser l'hydroxyle en position 3' du ribose par un groupement phosphorylé bien connu de l'homme du métier. De préférence, dans un premier mode de réalisation, dans le procédé de l'invention on utilise un groupe phosphoramidite, le plus préférablement un groupe 2-cyanoéthyl-*N,N*-diisopropylphosphoramidite.

Le composé de formule III suivante est alors obtenu : 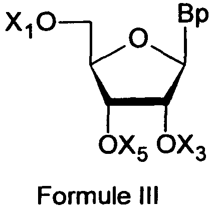 dans laquelle :
- Bp est une nucléobase naturelle ou modifiée, uracile ou une nucléobase adénine protégée ou une nucléobase cytosine protégée ou une nucléobase guanine protégée,
- X₁ est un groupe protecteur d'hydroxyle, choisi parmi des groupements acido-labiles et préférentiellement le groupe diméthoxytrityle,
- X₅ est un groupe phosphoramidite, de préférence un groupe 2-cyanoéthyl-*N,N*-diisopropylphosphoramidite,
- X₃ est un groupe de formule A telle que définie précédemment.

Mais, dans un second mode de réalisation, dans le procédé de l'invention, on utilise, pour la fonctionnalisation de l'hydroxyle en position 3', un groupe hydrogénophosphonate monoester.

Le composé de formule III qui est alors obtenu est un composé de formule III dans lequel Bp, X₁, et X₃ sont tels que définis précédemment mais X₅ est un groupe hydrogénophosphonate monoester.

A partir des monomères ribonucléotidiques de formule III et du monomère ribonucléotidique de formule II dans laquelle X₆ est OX₃ ou OAc ou désoxyribonucléotidique de formule II dans laquelle X₆ est H, un ARN simple brin protégé est synthétisé sur support solide.

Le procédé de synthèse de cet ARN simple brin protégé est également un objet de l'invention.

Cet ARN simple brin protégé a la formule I suivante : 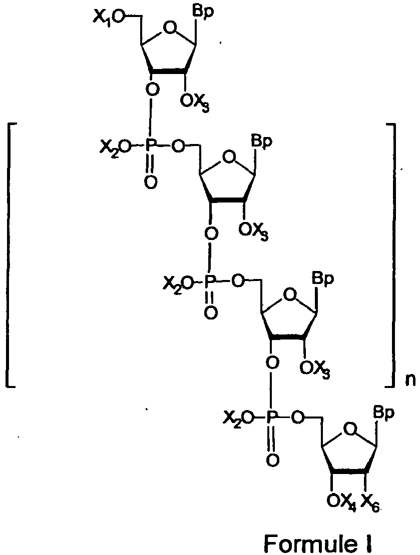 dans laquelle :
- X₁ est H ou un groupe protecteur d'hydroxyle choisi parmi des groupements acido-labiles et préférentiellement un groupe diméthoxytrityle,
- X₂ est H ou un groupe protecteur du phosphate, de préférence un groupe 2-cyanoéthyle,
- X₃ est un groupe de formule A suivante : 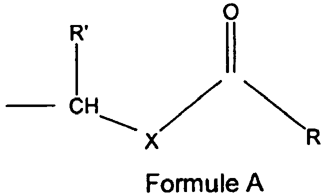 dans laquelle X est O ou S, R' est H ou CH₃, et R est choisi parmi un groupe alkyle en C₁ à C₄ linéaire ou ramifié et un groupe R₁-O-R₂ dans lequel R₁ est un groupe alkyle en C₁ à C₂ et R₂ est un groupe CH₃ ou CH₂CH₂-O-CH₃ ou aryle,
- X₄ représente l'ensemble du support solide et du lien qui relie l'oligonucléotide au support. Le support peut être des billes de verre (LCA-CPG : long chain alkylamine-controlled pore glass) ou des billes de résine polystyrène (HCP : highly cross-linked polystyrene). Le lien est choisi parmi des liens rompus par une base à la fin de l'élongation tels que oxalyle, succinyle, glyoxal, hydroquinone-*O,O*-diacetic acid (Q-linker) et préférentiellement le lien succinyle ou Q-linker ,
- X₆ est H ou un groupe OX₃ ou OAc,
- Bp est une nucléobase naturelle ou modifiée, thymine lorsque X₆ est H ou uracile lorsque X₆ est OX₃ ou OAc ou alors adénine protégée, ou cytosine protégée, ou guanine protégée, quelque soit X₆, et,
- n est un entier supérieur ou égal à 0.

Lorsque X₆ est OAc, les positions de X₄ et X₆ sont interchangeables.

Le procédé de synthèse selon l'invention de cet ARN simple brin comprend une étape de liaison d'un monomère ribonucléosidique ou désoxyribonucléosidique de formule II avec un support tel que défini précédemment, par l'intermédiaire d'un lien de préférence lui aussi baso-labile, suivie d'une étape d'assemblage d'un tel monomère de formule II avec au moins un monomère de formule III, et, optionnellement d'une étape de traitement acide de l'assemblage de monomères obtenu pour enlever le groupe protecteur X₁, enfin, une étape de libération de l'ARN protégé ainsi obtenu.

Ainsi, l'ARN sur lequel l'étape de libération est mise en oeuvre est soit un ARN de formule I dans laquelle X₁ est H lorsque l'étape de traitement acide est effectuée, soit un ARN de formule I dans laquelle X₁ est un groupe protecteur d'hydroxyle lorsque l'étape de traitement acide n'est pas effectuée.

L'assemblage des monomères ribonucléotidiques de formule III se fait généralement par synthèse automatisée sur un support solide.

Dans ce cas, l'ARN protégé synthétisé est lié, par son extrémité X₄ en position 3', par un lien au support solide.

Lorsque ce lien est également baso-labile, la libération de l'ARN de son support se fait, dans le procédé de l'invention, en même temps que la déprotection de l'OH en position 2' du ribose, et sans étape additionnelle.

Cela est vrai pour tout lien baso-labile.

Une fois l'ARN simple brin libéré, un ARN double brin peut être alors formé par hybridation de l'ARN simple brin libéré avec un ARN simple brin ayant une séquence complémentaire. L'ARN simple brin ayant une séquence complémentaire peut, lui aussi, avoir été synthétisé par le procédé de l'invention, ou encore avoir été synthétisé par un autre procédé.

Cela permet en particulier de pouvoir fabriquer des siARN.

Le procédé de libération de l'ARN simple brin protégé de formule I est un objet de l'invention. Ce procédé surmonte un préjugé de l'art antérieur en ce qu'il consiste à éliminer les groupes protecteurs des hydroxyles en positions 2' du ribose par un traitement basique.

Plus précisément, ce traitement basique comprend une étape a) de traitement de l'ARN protégé de formule I avec une base forte choisie parmi la pipéridine, la 1,8-diazabicyclo[5.4.0]undec-7-ène (DBU), la triéthylamine, à température ambiante, pour libérer le phosphate des liens internucléotidiques 3'-5'.

Cette étape a) n'est effectuée que lorsque la voie de synthèse aux phosphoramidites est utilisée.

En effet, lorsque la voie de synthèse aux hydrogénophosphonates est utilisée, l'assemblage des monomères mène à l'obtention de liens internucléosidiques 3'-5' hydrogénophosphonates diesters qui sont ainsi ensuite oxydés en phosphates pour obtenir un ARN, qui sera alors à libérer par le procédé de l'invention, de formule I dans laquelle X₂ est H.

Cette étape a) éventuelle est suivie d'une étape b) de traitement de l'ARN ainsi partiellement libéré obtenu à l'étape a), avec une base forte choisie parmi l'ammoniaque concentré, la méthylamine, le carbonate de potassium à température ambiante.

L'étape b) est elle commune pour la libération de l'ARN quelle que soit la voie de synthèse de l'ARN utilisée.

Le traitement avec la pipéridine ou la DBU à température ambiante se fait dans tout solvant approprié connu de l'homme du métier, et plus préférablement dans le THF ou l'acétonitrile sec, pendant 15 minutes (pipéridine) ou 45 minutes (DBU 0.45M) ou 1 minute (DBU 1 M).

Après cette déprotection du phosphate du lien internucléotidique 3'-5', les groupes protecteurs pivaloyloxyméthyle, *iso*butyryloxyméthyle, n-butyryloxyméthyle, propionyloxyméthyle, acétyloxyméthyle, selon le groupement utilisé, sont toujours en place.

Puis les groupes hydroxyles en position 2' sont libérés par traitement avec de l'ammoniac aqueux concentré, à température ambiante, pendant 3h. Aucune rupture des ARN ne se produit à cette étape. Cette déprotection séquentielle en deux étapes avec d'abord la DBU ou la pipéridine puis avec l'ammoniac aqueux est nécessaire pour éviter l'attaque par des hydroxyles en position 2' libérés, des phosphotriesters ou des phosphodiesters avoisinants, ce qui pourrait mener à une rupture de chaîne.

Cependant, lorsque l'ARN protégé de formule I contient les nucléobases naturelles ou modifiées : thymine ou uracile, adénine, cytosine et guanine c'est-à-dire au moins quatre monomères de formule III dans lesquels les nucléobases sont toutes différentes, le procédé de libération de l'invention comprend de plus une étape d'addition de 15% d'isopropylamine après le traitement avec l'ammoniaque.

Dans tous les cas, le milieu de déprotection est évaporé sous pression réduite, pour obtenir l'ARN libéré pur.

Afin de mieux faire comprendre l'invention, on va maintenant en décrire, à titre d'exemples purement illustratifs et non limitatifs plusieurs modes de mise en oeuvre.

Dans les exemples qui suivent, les monomères ribonucléotidiques de formule III avec X₅ est un groupe phosphoramidite, de préférence un groupe 2-cyanoéthyl-*N,N*-diisopropylphosphoramidite ont été synthétisés selon le schéma général suivant :

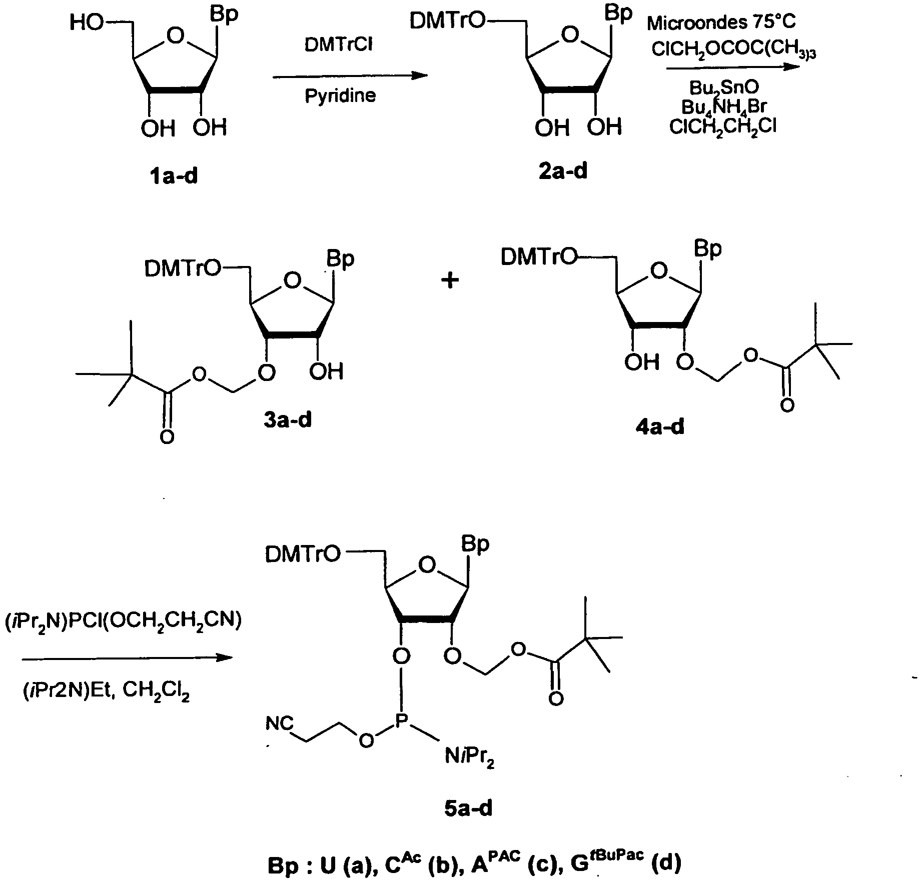

Dans ce schéma, la première étape de protection des bases guanine, cytosine et adénosine n'est pas indiquée.

Dans ce schéma, Bp peut être aussi G^{*i*PrPAC}, auquel cas, l'étape d'introduction du groupe pivaloyloxyméthyl en position 2' du composé 2a-d s'effectue sans traitement aux micro-ondes, comme cela sera expliqué ci-après.

### Exemple 1 : Synthèse du monomère 2'-O-pivaloyloxyméthyl-5'-O-(4,4'-diméthoxytrityle) uridine-3'-O-(2-cyanoéthyl-N,N-diisopropylphos-phoramidite 5a.

Le monomère synthétisé ici a la formule III dans laquelle la nucléobase est une nucléobase uracile, le groupe protecteur de l'hydroxyle en position 5' du ribose est le diméthoxytrityle, le groupe greffé sur l'hydroxyle en position 3' du ribose est le 2-cyanoéthyl-*N,N*-diisopropylphosphoramidite, et le groupe protecteur de l'hydroxyle en position 2' du ribose est le pivaloyloxyméthyle.

Pour cette synthèse, on démarre de la 5'-*O*-(4,4'-diméthoxytrityl)uridine **2a.** II n'y a donc plus qu'à introduire dans une première étape le groupement pivaloyloxyméthyle en position 2' du ribose et le groupe 2-cyanoéthyl-*N,N*-diisopropylphosphoramidite en position 3' du monomère.

Pour cela, le groupe pivaloyloxyméthyle est d'abord introduit en position 2' du ribose.

Plus précisément, on ajoute à une solution de 5'-*O*-(4,4'-diméthoxytrityl)uridine **2a** (2,5 g, 4,6 mmol, 1 eq) dans du dichloroéthane (DCE) (15 mL), du bromure de tétrabutylammonium (1,93 g, 5,98 mmol, 1,3 eq), de l'oxyde de dibutylétain (1,5 g, 5,98 mmol, 1,3 eq) et du chlorométhyl pivalate (1,67 mL, 11,5 mmol, 2,5 eq).

Le milieu réactionnel est chauffé sous irradiations micro-ondes (puissance 300 W) à 75°C pendant 2h30, refroidi et le solvant est évaporé.

Puis, le mélange réactionnel est soumis à une chromatographie sur colonne de gel de silice avec un gradient d'acétone (0-40%) dans du dichlorométhane. L'isomère éluant en premier est le composé désiré **4a.** Il est obtenu sous la forme d'une mousse blanche après évaporation du solvant.

Rendement **4a :** 1,1 g, 36%
RMN ¹H (300 MHz, HH-COSY, CDCl₃): δ 9,57 (s, 1 H, N*H*); 7,94 (d, J_{H-6/H}-₅ = 8.1 Hz, 1H, H-6); 7,37-7,09 (m, 9H, H ar, DMTr); 6,83-6,69 (m, 4H, H ar, DMTr); 5,87 (d, ³J_{H1'/H2'} = 1.8 Hz, 1H, *H*-1'); 5,52, 5,37 (2d_{AB}, J_{AB} = 6,3 Hz, 1H+1H, OC*H*₂O); 5,23 (d, J_{H-5/H-6} = 8,1 Hz, 1H, H-5); 4,39 (ddd, ³J_{H3'/OH3'} = 8,8 Hz; ³J_{H3'M4'}= 7,7 Hz; ³J_{H3'/H2'} = 5,2 Hz, 1H, *H*-3'); 4,24 (dd, ³J_{H2'/H3'} = 5,2 Hz; ³J_{H2'/H1'} = 1,8 Hz, 1H, *H*-2'); 3,95 (dt, ³J_{H4'/H3'} = 7.7 Hz; ³J_{H4'/H5',H5"} = 2,1 Hz, 1H, H-4'); 3,71 (s, 6H, 2 OC*H*₃); 3,48 (dd, ²J_{H5'/H5"} = 11,2 Hz; ³J_{H5'/H4'}= 2,1 Hz, 1H, H-5'); 3,42 (dd, ²J_{H5"/H5'} = 11,2 Hz; ³J_{H5"/H4'} = 2,1 Hz, 1H, H-5"); 2,48 (d, J_{OH-3'/H-3'} = 8,8 Hz, 1H, O*H*₃'); 1,15 (s, 9H, OCOC(CH₃)₃). RMN ¹³C (75 MHz, CDCl₃): δ 177,1 (OC=O); 162,5 (C=O); 157,7; 157,6; 143,3; 134,2; 133,9 (Cq, Car); 149,2 (C=O); 138,8 (C₆); 129,2; 129,1; 127,1; 127,0; 126,2; 112,3 (CH, Car); 101,2 (C₅); 87,2 (C_{1'}); 86,9 (OCH₂O); 86,1 (OCq, DMTr); 82,2 (C_{4'}); 81,1 (C_{2'}); 67,5 (C_{3'}); 60,1 (C_{5'}); 54,2 (OCH₃, DMTr); 37,8 (Cq, OCOC(CH₃)₃); 25,9 (OCOC(CH₃)₃). HRMS (FAB) calculé pour : C₃₆H₄₀N₂O₁₀ [M+H]⁺ 660,2709; trouvé : 660,2683.

A partir du 2'-*O*-pivaloyloxyméthyl-5'-*O*-(4,4'-diméthoxytrityl)uridine **4a** ainsi obtenu, le 2'-*O*-pivaloyloxyméthyl-5'-*O*-(4,4'-diméthoxytrityl)uridine 3'-*O*-(2-cyanoéthyl-*N,N*-diisopropylphosphoramidite **5a** a été obtenu de la façon suivante :

Le 2'-O-pivaloyloxyméthyl-5'-O-(4,4'-diméthoxytrityl)uridine obtenu à l'étape précédente **4a** (1g, 1,51 mmol, 1 eq) est séché par trois coévaporations avec du CH₃CN anhydre. Puis le résidu est dissout dans du CH₂Cl₂ anhydre (10 mL) et un mélange de *N,N*-diisopropyléthylamine (474 µL, 2,72 mmol, 1,8 eq), de 2-cyanoéthyl-*N,N*-diisopropylchlorophosphoramidite (506 µL, 2,27 mmol, 1,5 eq) et de CH₂CL₂ (1 mL) est ajouté goutte à goutte. Le mélange est agité sous argon, à température ambiante pendant 3h.

Après cela, de l'acétate d'éthyle est ajouté, le mélange réactionnel est versé dans une solution de NaHCO₃ saturé et des extractions avec AcOEt sont effectuées. Le mélange obtenu après séchage de l'extrait sur Na₂SO₄, élimination du solvant, est purifié par chromatographie sur colonne de gel de silice avec un gradient de CH₂Cl₂ (60-100%) dans du cyclohexane avec 1% de pyridine. Le monomère de formule III désiré **5a** est obtenu. II se présente sous la forme d'une mousse blanche, après évaporation du solvant.

Rendement **5a :** 1,05 g, 81%.
RMN ³¹P(121 MHz, CD₃CN): δ 150,35; 149,20.

### Exemple 2 : Synthèse du N⁴-acétyl-2'-O-pivaloxyoxyméthyl-5'-O-(4,4'-diméthoxytrityl) cytidine-3'-O-(2-cyanoéthyl-N,N-diisopropyl phosphoramidite 5b.

Ce monomère ribonucléotidique de formule III est le monomère dans lequel la nucléobase est la cytosine, le groupement protecteur de l'hydroxyle en position 5' du ribose est le diméthoxytrityle, le groupement greffé sur l'hydroxyle en position 3' du ribose est le 2-cyanoéthyl-*N,N*-diisopropylphosphoramidite et le groupement protecteur de l'hydroxyle en position 2' du ribose est le pivaloyloxyméthyle.

On part dans cette synthèse du *N*⁴-acétyl-5'-*O*-(4,4'-diméthoxytrityl) cytidine **2b,** ce qui signifie que cet exemple décrit la protection des OH en position 2' et 3' du ribose.

Ces protections ont été effectuées de la manière suivante :

A une solution de *N*⁴-acétyl-5'-*O*-(4,4'-diméthoxytrityl)cytidine **2b** (2,5 g, 4,26 mmol, 1 eq) dans du DCE (15 mL) est ajouté du bromure de tétrabutyl ammonium (1,78 g, 5,54 mmol, 1,3 eq), de l'oxyde de dibutylétain (1,39 g, 5,54 mmol, 1,3 eq), et du chlorométhyl pivalate (1,55 mL, 10,65 mmol, 2,5 eq). Le mélange réactionnel est chauffé sous irradiations micro-ondes (puissance 300 W) à 75°C pendant 2h30, refroidi et le solvant est évaporé.

Le mélange réactionnel est soumis à une chromatographie sur colonne de gel de silice avec un gradient d'acétone (40-100%) dans du dichlorométhane.

L'isomère éluant en premier est le composé désiré 4b qui est obtenu sous la forme d'une mousse blanche après évaporation du solvant.

Rendement **4b** : 1,14 g, 38%.
RMN ¹H (400 MHz, HH-COSY, CDCl₃): δ 9,84 (s, 1H, N*H*); 8,42 (d, J_{H-6/H-s5}= 7.5 Hz, 1H, *H*-6); 7,34-7,14 (m, 9H, H ar, DMTr); 7,06 (d, J_{H-5/H-6} = 7,5 Hz, 1H, H-5); 6,80-6,76 (m, 4H, H ar, DMTr); 5,86 (s, 1H, *H*-1'); 5,60; 5,50 (2d_{AB}, J_{AB} = 6,1 Hz, 1H+1H, OCH₂O); 4,34 (dt, ³J_{H3'/OH3'} = 10,6 Hz; ³J_{H3'/H4',H2'}= 5,2 Hz, 1H, *H*-3'); 4,21 (d, ³J_{H2'/H3'} = 5,2 Hz, 1H, *H*-2'); 3,98 (dt, ³J_{H4'/H3'} = 5,2 Hz; ³J_{H4'/H5',H5"}= 1,9 Hz, 1H, H-4'); 3,71 (s, 6H, 2 OCH₃); 3,52 (dd, ²J_{H5'/H5"} = 11,1 Hz; ³J_{H5'/H4'}= 1,9 Hz, 1H, H-5'); 3,48 (dd, ²J_{H5"/H5'} = 11,1 Hz; ³J_{H5"/H4'}= 1,9 Hz, 1H, *H*-5"); 2,50 (d, J_{OH-3'/H-3'} = 10,6 Hz, 1H, O*H*₃'); 2,19 (s, 3H, NHCOCH3); 1,13 (s, 9H, OCOC(C*H*₃)₃). RMN ¹³C (100 MHz, CDCl₃): δ 178,2 (OC=O); 170,6 (NHCO); 163,1 (C₄); 158,7 (Cq, Car); 147,4 (C₂); 146,6 (C₆); 146,3; 135,2 (Cq, Car); 130,1; 129,2; 128,2; 128; 127,9; 127,8; 127,2; 127; 113,3; 113,1 (CH, Car); 96,8 (C₅); 91,5 (C_{1'}); 88,0 (OCH₂O); 87,6 (OCq, DMTr); 83,1 (C_{4'}); 81,7 (C_{2'}); 67,8 (C_{3'}); 60,7 (C_{5'}); 55,3 (OCH₃, DMTr); 38,9 (Cq, OCOC(CH₃)₃); 27,0 (OCOC(CH₃)₃); 18,1 (COCH3). HRMS (FAB) calculé pour C₃₈H₄₄N₃O₁₀ [M+H]⁺: 702.3042; trouvé : 702,3027.

Puis, 1,3 g, 1,85 mmol, 1 eq du composé ainsi obtenu **4b** sont séchés par trois coévaporations avec du CH₃CN anhydre. Le résidu obtenu est dissout dans du CH₂Cl₂ anhydre (13 mL) et un mélange de *N,N-*diisopropyléthylamine (580 µL, 3,33 mmol, 1,8 eq) de 2-cyanoéthyl-*N,N-*diisopropylchlorophosphoramidite (620 µL, 2, 78 mmol, 1,5 eq) et de CH₂Cl₂ (1 mL) est ajouté goutte à goutte. Le mélangé est agité sous argon, à température ambiante pendant 3h. Après cela, de l'acétate d'éthyle est ajouté, le mélange réactionnel est versé dans une solution de NaHCO₃ saturée et des extractions avec AcOEt sont effectuées. Le mélange obtenu après séchage de l'extrait sur Na₂SO₄, élimination du solvant, est purifié par chromatographie sur colonne de gel de silice avec un gradient de AcOEt (10-80%) dans de l'hexane avec 1% de pyridine.

Le monomère ribonucléotidique de formule III voulu 5b est obtenu sous la forme d'une mousse blanche après évaporation du solvant.

Rendement **5b :** 1,37 g, 82%.
RMN ³¹P(121 MHz, CD₃CN): δ 150,15; 148,35.

### Exemple 3 : Synthèse du N⁶-Phénoxyacétyl-2'-O-pivaloyloxyméthyl-5'-O-(4,4'-diméthoxytrityl) adénosine 3'-O-(2-cyanoéthyl-N,N-diisopropylphosphoramidite) 5c.

Ce composé correspond au monomère de formule III dans lequel la nucléobase est l'adénine dont l'amine est protégée par un groupement phénoxyacétyle, le groupement protecteur de l'hydroxyle en position 5' du ribose est le diméthoxytrityle, le groupement greffé sur l'hydroxyle en position 3' est le 2-cyanoéthyl-*N,N*-diisopropylphosphoramidite, et le groupement protecteur de l'hydroxyle en position 2' du ribose est le pivaloyloxyméthyle.

Pour cette synthèse, on part du ribonucléoside **2c** dont la nucléobase et l'hydroxyle en position 5' sont protégés.

A une solution de *N*⁶-phénoxyacétyl-5'-*O*-(4,4'-diméthoxytrityl) adénosine **2c** (1g, 1,42 mmol, 1eq) dans du DCE (5 mL) est ajouté du tétrabutylammonium bromure (596 mg, 1,85 mmol, 1,3 eq) du dibutylétain oxyde (462 mg, 1,85 mmol, 1,3 eq) et du chlorométhyl pivalate (515 µL, 3,55 mmol, 2,5 eq). Le mélange réactionnel est chauffé sous irradiations micro-ondes (puissance 300 W) à 75°C pendant 2h30, refroidi et le solvant est évaporé. Le mélange réactionnel est soumis à une chromatographie sur colonne de gel de silice avec un gradient d'acétone (0-30%) dans du dichlorométhane. L'isomère éluant en premier est le composé désiré **4c,** sous la forme d'une mousse blanche après évaporation du solvant.

Rendement **4c :** 395 mg, 34%.
RMN ¹H (400 MHz, HH-COSY, CDCl₃): δ 9,50 (s, 1H, N*H*); 8,72 (s, 1H, *H*-2); 8,25 (s, 1H, *H*-8); 7,44-6,81 (m, 18H, H ar, DMTr-Pac); 6,22 (d, ³J_{H1'/H2'} = 4,7 Hz, 1H, H-1'); 5,51; 5,40 (2d_{AB}, J_{AB} = 5,8 Hz, 1H+1H, OC*H*₂O); 5,08 (t, ³J_{H2'/H3',H1'} = 4,7 Hz; 1H, H-2'); 4,88 (s, 2H, NHCOC*H*₂Ph); 4,55 (q, ³J_{H3'/H2',OH3',H4'} = 4,7 Hz, 1H; *H*-3'); 4,28 (m, 1H, *H*-4'); 3,79 (s, 6H, 2 OC*H*₃); 3,54 (dd, ²J_{H5'/H5"} = 10,7 Hz; ³J_{H5'/H4'}= 3,3 Hz, 1H, *H*-5'); 3,43 (dd, ²J_{H5"/N5'} = 10,7 Hz; ³J_{H5"/H4'}= 4 Hz, 1H, *H*-5"); 2,75 (d, J_{OH-3'/H-3'} = 4,7 Hz, 1H, O*H*₃'); 1,17 (s, 9H, OCOC(C*H*₃)₃). RMN ¹³C (100 MHz, CDCl₃): δ 177,9 (OC=O); 166,7 (NHCO); 158,6 (Cq, Car); 157,2 (Cq, Pac); 152,6 (C₂); 151,5 (C₆); 148,4 (C₄); 144,4; 135,5 (Cq, Car); 142,2 (C₈); 130,1; 129,9; 128,1; 127,9; 122,4; 115; 114,9; 113,2 (CH, Car); 123,2 (C₅); 89,0 (OCH₂O); 87,3 (C_{1'}); 86,7 (OCq, DMTr); 84,2 (C_{4'}); 81,7 (C_{2'}); 70,5 (C_{3'}); 68,1 (NHCOCH₂Ph); 62,9 (C_{5'}); 55,3 (OCH₃, DMTr); 38,9 (Cq, OCOC(CH₃)₃); 27,0 (OCOC(CH₃)₃). HRMS (FAB) calculé pour C₄₅H₄₈N₅O₁₀ [M+H]⁺ : 818,3401; trouvé : 818,3397.

Le composé obtenu **4c** (550 mg, 0,67 mmol, 1 eq) est séché par trois coévaporations avec CH₃CN anhydre. Puis le résidu est dissout dans du CH₂Cl₂ anhydre (7 mL) et un mélange de *N,N*-diisopropyléthylamine (211 µL, 1,21 mmol, 1,8 eq), de 2-cyanoéthyl-*N,N*-diisopropylchlorophosphoramidite (223 µL, 1 mmol, 1,5 eq) et de CH₂Cl₂ (0,5 mL) est ajouté goutte à goutte. Le mélange est agité sous argon, à température ambiante pendant 2h. Après cela, de l'acétate d'éthyle est ajouté, le mélange réactionnel est versé dans une solution saturée de NaHCO₃ et des extractions avec AcOEt sont effectuées.

Le mélange obtenu après séchage de l'extrait sur Na₂SO₄ et élimination du solvant, est purifié par chromatographie sur colonne de gel silice avec un gradient de CH₂Cl₂ (40-100%) dans du cyclohexane avec 1% de pyridine. Le monomère ribonucléotidique désiré 5c est obtenu sous la forme d'une mousse blanche après évaporation du solvant.

Rendement 5c : 540 mg, 79%.
RMN ³¹P (121 MHz, CDCl₃):δ150,85; 150,80.

### Exemple 4 : Synthèse du N²-tert-butylphénoxyacétyl-2'-O-pivaloyloxyméthyl-5'-O-(4,4'-diméthoxytrityl) guanosine 3'-O-(2-cyanoéthyl-N,N-diisopropylphosphoramidite) 5d.

Le composé synthétisé dans cet exemple est le monomère de formule III 5d dans lequel la nucléobase est la guanine protégée par du *tert-*butylphénoxyacétyle, l'hydroxyle en position 5' est le diméthoxytrityle, le groupement greffé sur l'hydroxyle en positon 3' est le 2-cyanoéthyl-*N*,*N-*diisopropylphosphoramidite, et le groupement protecteur de l'hydroxyle en position 2' du ribose est le pivaloyloxyméthyle.

On part ici du ribonucléoside 2d dont la nucléobase ainsi que le groupement hydroxyle en position 5' du ribose sont protégés.

A une solution de ***N²-tert* butylphénoxyacétyl-5'-*O*-(4,4'-**diméthoxytrityl) guanosine **2d** (2,6 g, 3,35 mmol, 1 eq) dans du DCE (15 mL) est ajouté du tétrabutylammonium bromure (1,4 g, 4,36 mmol, 1,3 eq), de l'oxyde de dibutylétain (1,1 g, 4,36 mmol, 1,3 eq), et du chlorométhyl pivalate (1,22 mL, 8,38 mmol, 2,5 eq). Le mélange réactionnel est chauffé sous irradiations micro-ondes (puissance 300 W) à 75°C pendant 2h, refroidi et le solvant est évaporé. Le mélange réactionnel est soumis à une chromatographie sur colonne de gel de silice avec un gradient d'acétone (0-15%) dans du dichlorométhane. L'isomère éluant en premier est le composé désiré **4d** qui est obtenu sous la forme d'une mousse blanche après évaporation du solvant.

Rendement **4d :** 1,46 g, 49%.
RMN ¹H (400 MHz, HH-COSY, CDCl₃): 8 11,78 (s, 1H, N*H*-1); 9,09 (s, 1H, N*H*_{Pac}); 7,79 (s, 1H, *H*-8); 7,36-6,73 (m, 18H, H ar, DMTr-*tbu*Pac); 5,99 (d, ³J_{H1'/H2'} = 4,7 Hz, 1H, *H*-1'); 5,46; 5,36 (2d_{AB}, J_{AB} = 5,3 Hz, 1H+1H, OC*H*₂O); ); 5,23 (s, 2H, NHCOC*H*₂Ph); 4,65 (t, ³J_{H2'/H3',H1'} = 4,7 Hz; 1H, H-2'); 4,34 (m, 1H, H-3'); 4,17 (m, 1H, H-4'); 3,70 (s, 6H, 2 OC*H*₃); 3,38 (dd, ²JH_{5'/H5"} = 10,7 Hz; ³J_{H5'/H4'}= 2,7 Hz, 1H, *H*-5'); 3,35 (dd, ²J_{H5"/H5'} = 10,7 Hz_{;} ³J_{H5"/H4'}= 2,9 Hz, 1H, *H*-5"); 2,49 (d, J_{OH-3'/H-3'} = 4,3 Hz, 1H, O*H*₃'); 1,24 (s, 9H, OCOC(C*H*₃)₃); 1,10 (s, 9H, *tbu*Pac)*.* RMN ¹³C (100 MHz, CDCl₃): δ 177,9 (OC=O); 169,9 (NHCO); 158,6 (Cq, Car); 155,2 (C₆); 154,2 (Cq, *tbu*Pac); 147,7 (C₂); 146,5 (C₄); 145,9 (Cq, *tbu*Pac); 144,3; 135,5 (Cq, Car); 137,0 (C₈); 130,1; 128,1; 127,1; 126,9; 126,8; 114,4; 113,3 (CH, Car); 122,3 (C₅); 89,3 (OCH₂O); 86,8 (OCq, DMTr); 86,1 (C_{1'}); 84,0 (C_{2'}); 82,7 (C_{4'}); 70,4 (C_{3'}); 67,1 (NHCOCH₂Ph); 63,1 (C_{5'}); 55,3 (OCH₃, DMTr); 38,9 (Cq, OCOC(CH₃)₃); 34,3 (Cq, PhC(CH₃)₃); 31.4 (PhC(CH₃)₃); 27 (OCOC(CH₃)₃). HRMS (ESI) calculé pour C₄₉H₅₆N₅O₁₁ [M+H]⁺ : 890,3976; trouvé : 890,3934.

Le composé obtenu **4d** (700 mg, 0,79 mmol, 1 eq.) est séché par trois coévaporations avec du CH₃CN anhydre. Puis le résidu est dissout dans du CH₂Cl₂ anhydre (5 mL) et un mélange de *N*,*N*-diisopropyléthylamine (247 µL, 1,42 mmol, 1,8 eq.), de 2-cyanoethyl-*N*,*N*-diisopropylchlorophosphoramidite (265 µL, 1,19 mmol, 1,5 eq.) et de CH₂Cl₂ (0,5 mL) est ajouté goutte à goutte. Le mélange est agité sous argon, à température ambiante pendant 3 h. Après cela, de l'acétate d'éthyle est ajouté, le mélange réactionnel est versé dans une solution saturée de NaHCO₃ et des extractions avec AcOEt sont effectuées.

Le mélange obtenu après séchage de l'extrait sur Na₂SO₄ et élimination du solvant, est purifié par chromatographie sur colonne de gel de silice avec un gradient de CH₂Cl₂ (40-100%) dans du cyclohexane avec 1% de pyridine. Le monomère ribonucléotidique désiré **5d** est obtenu sous la forme d'une mousse blanche après évaporation du solvant.

Rendement **5d** : 646 mg, 75%.
NMR ³¹P (121 MHz, CD₃CN): δ 150.43, 149.67.

Le procédé décrit dans les exemples qui précèdent pour obtenir les composés **4a-d,** et correspondant à la réaction d'introduction du groupement pivaloyloxyméthyle, implique l'utilisation d'un appareil à micro-ondes. Cette utilisation peut être un frein à sa transposition à grande échelle pour la production des ribonucléotides et le dévéloppement de la méthodologie de la synthèse d'ARN par le procédé de l'invention. Pour cette raison, l'invention propose également un procédé de synthèse des composés 5 ci-dessus ne comportant pas d'utilisation de micro-ondes. Dans ce procédé, d'une part, la nucléobase Bp est protégée par un groupe *i*PrPAC et, d'autre part, l'étape d'introduction du groupe pivaloyloxyméthyle pour obtenir les composés **4** a été remplacée par la procédure générale suivante:

Les nucléosides **2a-d** sont solubilisés dans du DCE ou de l'acétonitrile anhydre, puis à température ambiante, sous agitation et sous argon sont ajoutés l'oxyde de dibutylétain (DBTO) puis le bromure de tétrabutylammonium (TBAB) puis le chlorométhylpivalate ou le iodométhylpivalate selon la nature du nucléoside. Le milieu réactionnel est alors chauffé et maintenu à 70°C ou 75°C sous argon pendant des temps variant de 1 h30 à 6h.

Par exemple, pour les composés dans lesquels Bp est G, on procède de la façon suivante :
**Synthèse du monomère 2'-*O*-pivaloyloxyméthyl-5'-*O*-(4,4'-diméthoxytrityle) uridine 4a** :
**Nucléoside** (1 eq), DBTO (1.3 eq), TBAB (1.3 eq), chlorométhylpivalate (2.5 eq) dans acétonitrile à 70°C pendant 3 h.
**Synthèse du *N*⁴-acétyl-2'-*O*-pivaloxyoxyméthyl-5'-*O*-(4,4'-diméthoxytrityl) cytidine 4b**
Nucléoside (1 eq), DBTO (1.5 eq), TBAB (1.5 eq), chlorométhylpivalate (3 eq) dans DCE à 75°C pendant 6 h.
**Synthèse du *N*⁶-Phénoxyacétyl-2'-*O*-pivaloyioxyméthyl-5'-*O*-(4,4'-diméthoxytrityl) adénosine 4c**
Nucléoside (1 eq), DBTO (1.3 eq), TBAB (1.3 eq), iodométhylpivalate (2.5 eq) dans DCE à 70°C pendant 1 h 30.
**Synthèse du *N*²-*iso*-propylphénoxyacétyl-2'-*O*-pivaloyloxyméthyl-5'-*O*-(4,4'-diméthoxytrityl) guanosine 4e.**

Le composé **4e** correspond au composé **4d** décrit au schéma général mais dans lequel Bp est protégée par le groupe *i*PrPAC.

Nucléoside (1 eq), DBTO (1.3 eq), TBAB (1.3 eq), chlorométhylpivalate (3 eq) dans DCE à 75°C pendant 3 h.

### Exemple 5 : Synthèse d'ARN simple brin de formule I par la chimie des phosphoramidites avec X₃ qui est un groupe pivaloyloxyméthyle.

A partir des monomères obtenus **5a-d** aux exemples 1 à 4, des ARN simple brin ont été synthétisés.

Ces ARN protégés de formule I ont été préparés sur un synthétiseur d'ADN ABI modèle 381A à une échelle de 1 µmol, en utilisant les monomères **5a-d** de formule III obtenus aux exemples 1 à 4, dans les conditions montrées au tableau 1 suivant.

**Tableau 1**

| Étape | Opération | Réactif | Temps (s) |
|---|---|---|---|
| 1 | Déblocage | 3% DCA dans CH₂Cl₂ | 60 |
| 2 | Couplage | 0,1 M amidite dans CH₃CN + 0.3 M BMT dans CH₃CN | 180* |
| 3 | Masquage | (Pac)₂O dans THF/pyridine + 10% NMI dans THF | 200 |
| 4 | Oxydation | 0,1 M I₂ dans THF/H₂O/Pyr | 20 |

| | | | |
|---|---|---|---|
| DCA = acide dichloroacétique BMT = benzylmercaptotétrazole (Pac) ₂O = anhydride phénoxyacétique NMI = *N*-méthylimidazole | | | |

• Un temps de couplage de 300 secondes a également été appliqué pour la synthèse des hétéropolymères mais n'améliore pas significativement le rendement de couplage.

Les ARN protégés ON1 à ON5 (SEQ ID NO :1 à SEQ ID NO :5) ont été synthétisés en utilisant du 5-benzylmercaptotétrazole (BMT) en tant qu'activateur, une solution d'iode en tant qu'agent oxydant et un mélange d'anhydride phénoxyacétique (Pac)₂O dans un mélange de THF/pyridine et N-méthylimidazole (NMI) dans du THF en tant que solution de masquage.

Après la déprotection dans les conditions décrites ci-dessus, les oligonucléotides correspondants aux ARN, déprotégés, ont été analysés par RP-HPLC (Dionex DX 600) avec une colonne nucléosil 100-5 C₁₈ (150 x 4,6 mm; Macherey-Nagel), par MALDI-TOF MS (Voyager Perpsective Biosystems) et finalement purifiés par HPLC avec une colonne Delta-Pak (7,8 X 300 mm 15µ C₁₈ 100Å; Waters).

Les ARN notés ON1 à ON5 ont été obtenus et avaient les formules montrées au tableau 2 suivant :

**Tableau 2**

| ON^{[a]} SEQ ID NO | 5'-séquence-3' | CT^{[b]} | OY^{[c]} | AY^{[d]} | Matériau brut^{[e]} |
|---|---|---|---|---|---|
| 1 | U₁₂dC | 180 | 96,5 | 99,7 | n.d^{.[f]} |
| 2 | U₁₉TT | 180 | 94,2 | 99,7 | 140 |
| 3 | CCC GUA GCU GTT | 180 | 91,1 | 99,1 | 86 |
| 4 | UGC AUC CUC GAU GGU AAC GdCT | 300 | 82,1 | 99,0 | 130 |
| 5 | CGU UAC CAU CGA GCA UCC AdAT | 300 | 83,8 | 99,1 | 125 |

| | | | | | |
|---|---|---|---|---|---|
| [a] ON = oligoribonucléotides [b] = temps de couplage (s) dans le cycle de synthèse automatisé [c] OY = rendement global de couplage (%) [d] AY = rendement de couplage moyen par étape [e] = matériau brut global (Unités D.O = densité optique) mesuré à 260 nm par absorption UV [f] n.d = non déterminé. | | | | | |

### Exemple 6 : Synthèse des monomères 2'-O-acyloxyméthyl-5'-O-(4,4'-diméthoxytrityl) uridine-3'-O-(2-cyanoéthyl-N,N-diisopropylphos-phoramidite 12a-d.

Dans cet exemple, les monomères ribonucléotidiques de formule III dans lesquels la nucléobase est l'uracile, le groupe protecteur de l'hydroxyle en position 5' du ribose est un groupe diméthoxytrityle et le groupe protecteur de l'hydroxyle en position 2' du ribose est soit un groupe *i*sobutyryloxyméthyle (composé **12a :** 2'-*O*-*i*sobutyryloxyméthyl-3'-*O*-(2-cyanoéthyl-*N*,*N*-diisopropylphosphoramidite)-5'-*O-*(4,4'-diméthoxytrityl)uridine))), soit un groupe butyryloxyméthyle (composé **12b** : 2'-*O*-butyryloxyméthyl-3'-*O*-(2-cyanoéthyl-*N*,*N*-diisopropylphosphoramidite)-5'-*O-*(4,4'-diméthoxytrityl)uridine, soit un groupe propionyloxyméthyle (composé **12c :** 2'-*O*-propionyloxyméthyl-3'-*O*-(2-cyanoéthyl-*N*,*N*-diisopropylphosphoramidite)-5'-*O*-(4,4'-diméthoxytrityl)uridine ))), soit un groupe acétyloxyméthyle : (composé **12d:** 2'-*O*-acétyloxyméthyl-3'-*O*-(2-cyanoéthyl-*N,N-*diisopropylphosphoramidite)-5'-*O*-(4,4'-diméthoxytrityl)uridine ont été synthétisés selon le schéma général suivant :

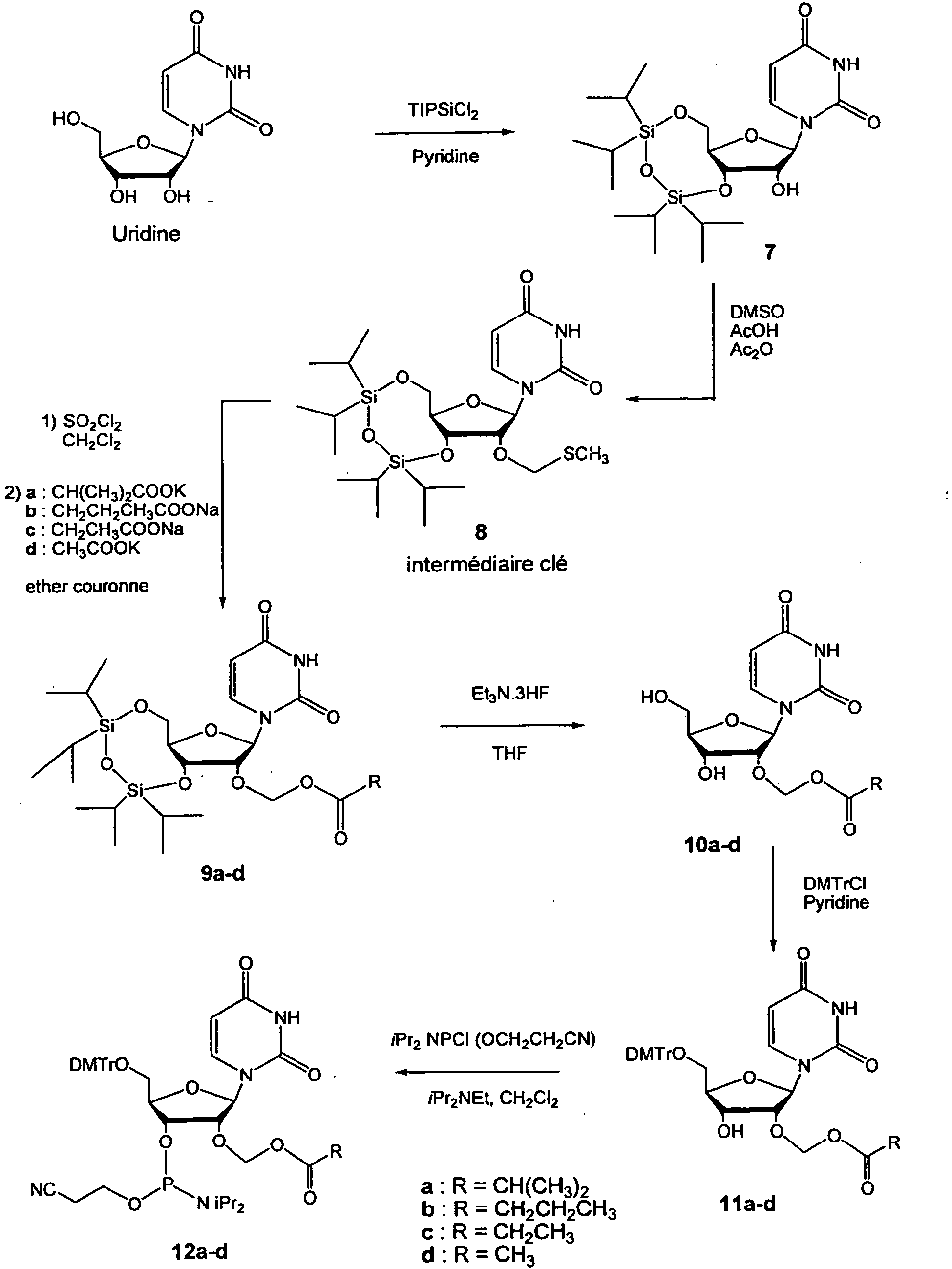

La première étape de cette synthèse consiste à préparer respectivement les composés **9a** à **9d** suivants :
2'-*O*-*iso*butyryloxyméthyl-3',5'-*O*-(tétraisopropyldisiloxane-1,3-diyl)uridine **(9a),**
2'-*O*-butyryloxyméthyl-3',5'-*O*-(tétraisopropyldisiloxane-1,3-diyl)uridine **(9b),**
2'-O-propionyloxyméthyl-3',5'-O-(tétraisopropyldisiloxane-1,3-diyl)uridine **(9c),**
2'-O-acétyloxyméthyl-3',5'-O-(tétraisopropyldisiloxane-1,3-diyl)uridine **(9d).**

On procède de la façon suivante :

A une solution de 2'-*O*-méthylthiométhyl-3',5'-*O-*(tétraisopropyldisiloxane-1,3-diyl)uridine **8** (3,07g, 5,61 mmol, 1eq) dans du CH₂Cl₂ (25 mL) a été ajouté goutte à goutte, sous argon, une solution de chlorure de sulfuryle 1,0M dans du CH₂Cl₂ (7,0 mL, 7,0 mmol, 1,25 eq). Le mélange réactionnel a été agité à température ambiante pendant 2 heures. Après la fin de la réaction, le dérivé chlorométhyl éther a été obtenu sous la forme d'une mousse brune après évaporation du solvant et a été directement utilisé dans l'étape suivante.

A une solution du dérivé chlorométhyl éther dans du CH₂Cl₂ (20 mL) a été ajouté goute à goutte une solution de potassium *i*sobutyrate (1,22 g, 9,60 mmol, 1,72 eq), ou de sodium butyrate (1,06 g, 9,60 mmol, 1,72 eq) ou de sodium propionate (922 mg, 9,60 mmol, 1,72 eq), ou de potassium acétate (926 mg, 9,60 mmol, 1,72 eq) et du dibenzo éther couronne-18-6 (1,48 g, 4,17 mmol, 0,75 eq) dans du CH₂Cl₂ (10mL) ou de l'éther couronne 15-5 (920 mg, 4,17 mmol, 0,75 eq), selon le cation. Après agitation à température ambiante pendant 3 heures, le mélange a été dilué avec de l'acétate d'éthyle et lavé avec de l'eau. Une extraction avec de l'AcOEt a été mise en oeuvre et l'extrait a été séché sur Na₂SO₄. Après concentration du solvant, le précipité éther couronne (seulement le dibenzo éther couronne 18-6) a été éliminé par filtration et le filtrat a été évaporé. Le mélange réactionnel a été soumis à une chromatographie sur colonne de gel de silice avec un gradient d'AcOEt (0-50%) dans du cyclohexane. Les composés voulus 9a, 9b, 9c et 9d ont été obtenus sous forme de mousses blanches après évaporation du solvant.
**9a.** (2,30 g, 70%). RMN ¹H (400 MHz, HH-COSY, CDCl₃) : 8,81 (s, 1H, N*H*) ; 7,80 (d, *J* _{H-6/H-5} = 8,1 Hz, 1H, *H*-6) ; 5,70 (s, 1*H*, H-1') ; 5,62 (d, *J* _{H-5/H-6}= 8,1 Hz, 1H, *H-*5) ; 5,45 (2d_{AB}, *J*_{AB} = 6,5 Hz, 2H, OC*H*₂O) ; 4,25-4,14 (m, 3H, *H*-2', *H*-3', *H*-5a') ; 4,06 (dd, *J* = 1,8 Hz, *J* = 9,5 Hz, 1H, *H*-4') ; 3,90 (dd, *J* _{H5'b/H4'} = 2,4 Hz, *J* = 13,6 Hz, 1H, *H*-5'b ) ; 2,54 (hept, *J* = 7,0 Hz , 1H, C(O)C*H*) ; 1,18-0,83 (m, 22H, iPr).
   RMN ¹³C (100 MHz, CDCl₃) : 176,6 (OC=O) ; 163,6 (C=O) ; 149,6 (C=O) ; 139,4 (C-6) ; 101,6 (C-5) ; 89,1 (C-1') ; 87,6 (OCH₂O) ; 81,7 (C-4') ; 81,3 (C-2') ; 67,8 (C-3') ; 59,3 (C-5') ; 33,9 (C(O)CH) 18,8-18,7-17,5-17,6-17,4-17,3-17,2-17,1-16,9-16,8 (CH₃, iPr) ; 13,4-13,2-13,1-12,9-12,5 (CH, iPr).
9b. (2.90 g, 89%). RMN ¹H (400 MHz, HH-COSY, CDCl₃) : 8,80 (s, 1H. N*H*) ; 7,79 (d, *J* _{H-6H-5} = 8,1 Hz, 1H, *H*-6) ; 5,66 (s, 1H, *H*-1') ; 5,61 (dd, *J* _{H-5/H-6} = 8,1 Hz, *J* _{H-5/NH} = 2,1 Hz, 1H, H-5) ; 5,46 (s, 2H, OC*H*₂O) ; 4,19-4,13 (m, 3H, *H*-2', *H*-3', *H*-5a') ; 4,06 (dd, *J* _{H-4'/H-5'b} = 1,8 Hz, *J* = 9,2 Hz, 1H, *H*-4') ; 3,90 (dd, *J* _{H-5'b/H-4'} = 2,3 Hz, *J* = 13,6 Hz, 1H, *H*-5'b); 2,28 (td, *J* = 7,4 Hz, *J* = 1,3 Hz , 2H, C(O)C*H*₂) ; 1,60 (sext, *J* = 7,4 Hz, 2H, C*H*_{2β}) ; 1,03-0,92 (m, 28H, iPr) ; 0,89 (t, *J* = 7,4 Hz, 3H, C*H*_{3γ}).
   RMN ¹³C (100 MHz, CDCl₃) : 173,2 (OC=O) ; 163,3 (C=O) ; 149,7 (C=O) ; 139,4 (C-6) ; 101,6 (C-5) ; 89,2 (C-1') ; 87,5 (OCH₂O) ; 81,7 (C-4') ; 81,3 (C-2') ; 67,8 (C-3') ; 59,3 (C-5') ; 36,1 (C(O)CH₂) ; 18,1 (CH_{2β}) ; 17,5-17,4-17,3-17,2-17,1-17,.0-16,9-13,6-13,4-13,0-12,9-12,5 (iPr, TIPS and CH_{3γ}).
9c. (2,37 g, 77%). RMN ¹H (400 MHz, HH-COSY, CDCl₃) : 9,18 (s, 1H, N*H*) ; 7,80 (d, *J* _{H-6/H-5} = 8,2 Hz, 1H, *H*-6) ; 5,67 (s, 1H, *H*-1') ; 5,61 (dd, *J* _{H-5/H-6} = 8,1 Hz, *J* _{H-5/NH} = 1,6 Hz, 1H, *H*-5) ; 5,45 (s, 2H, OCH₂O) ; 4,20-4,13 (m, 3H, *H*-2', *H*-3', *H*-5a') ; 4,06 (dd, *J* _{H-4'/H-S'b} = 1,7 Hz, *J* = 9, Hz, 1H, *H*-4') ; 3,90 (dd, *J* _{H-5'b/H-4'} = 2,1 Hz, J = 13,6 Hz, 1H, *H*-5'b ) ; 2,34 (q, *J* = 7.6 Hz, 2H, C(O)C*H*₂) ; 1,08 (t, *J* = 7,5 Hz, 3H, C*H*_{3β}) ; 1.,3-0,88 (m, 28H, iPr).
   RMN ¹³C (100MHz, CDCl₃) : 174,0 (OC=O) ; 163,6 (C=O) ; 149,8 (C=O) ; 139,3 (C-6) ; 101,6 (C-5) ; 89,2 (C-1') ; 87,5 (OCH₂O) ; 81,7 (C-4') ; 81,3 (C-2') ; 67,7 (C-3') ; 59,3 (C-5') ; 27,5 (C(O)C*_{α}*) ; 17,5-17,4-17,3-17,2-17,1-17,.0-16,8-13,4-13,0-12,9-12,5 (iPr, TIPS) ; 8.8 (CH_{3β}).
**9d.** L'identification de ce composé est identique à celle décrite pour le même composé dans Parey et al, "First Evaluation of Acyloxymethyl or Acylthiomethyl Groups as Biolabile 2'-O-Protections of RNA, Organic Letters", 2006, Vol.8, No.17, 3869-3872.

Puis les composés 2'-*O*-*is*obutyryloxyméthyl-uridine **10a,** 2'-*O-*butyryloxyméthyl-uridine **10b,** 2'-*O*-propionyloxyméthyl-uridine **10c** and 2'-*O-*acétyloxyméthyl-uridine **10d** ont été synthétisés de la manière suivante :

A une solution de **9a** (2,30 g, 3,92 mmol, 1 eq) ou **9b** (2,90 g, 4,94 mmol, 1 eq) ou **9c** (2,37 g, 4,41 mmol, 1 eq) ou 9d (2,90 g, 5,19 mmol, 1 eq), une solution d'Et₃N.3HF (pour **9a :** 767 µL, 15,68 mmol, 4 eq; pour **9b :** 970 µL, 19,80 mmol, 4 eq; pour **9c :** 865 µL, 17,64 mmol, 4, eq et pour **9d :** 1017 µL, 20,76 mmol, 4 eq) a été ajoutée. Après agitation entre 1 h30 à 5h à température ambiante, la déprotection était complète et le mélange réactionnel a été traité avec un tampon triéthylammoniumacétate (2M, pH 7), puis évaporé. Le mélange brut a été purifié par chromatographie sur gel de silice avec un gradient de MeOH (0-4,5%) dans CH₂Cl₂. Les composés voulus **10a** à **10d** ont été obtenus sous forme de poudres blanches après une lyophilisation avec du dioxane.
**10a.** (1,25 g, 93%). RMN ¹H (400 MHz, HH-COSY, DMSO): 11,38 (s, 1H, N*H*) ; 7,2 (d, *J* _{H-6/H-5}= 8,2 Hz, 1H, *H*-6) ; 5,89 (d, *J* _{H-1'/H-2'} = 5,4 Hz, 1H, *H*-1') ; 5,68 (d, *J* _{H}-_{5/H}-₆ = 8,0 Hz, 1H, *H*-5) ; 5,37, 5.,3 (2d_{AB}, *J*_{AB} = 6,5 Hz 2H, OC*H*₂O) ; 5,32 (s, 1H, O*H*-3') ; 5,20 (s, 1H, OH-5') ; 4,26 (t, *J* = 5,2 Hz, 1H, *H*-2') ; 4,14 (s, 1H, *H*-3') ; 3,88 (dd, *J* _{H-4'/H-5'b} = 3,0 Hz, *J* = 6,9 Hz, 1H, *H*-4'); 3,65 (dd, *J* _{H-5'a/H}-_{4'} = 2,3 Hz, *J* _{H-5'a/H-5'b} = 11,9 Hz, 1H, *H*-5'a) ; 3,57 (dd, *J* _{H-5'bH-4'}= 2,5 Hz, *J* _{H-5'b/H-5'a} = 11,8 Hz, 1H, *H*-5'b) ; 2,50 (hept, *J* _{CH/CH3}= 7,0 Hz, 1H, C(O)C*H*) ; 1,07 (d, *J* _{CH3/CH} = 7,0 Hz, 3H, CH₃), 1,06 (d, *J* _{CH3/CH} = 7.0 Hz, 3H, CH₃).
   RMN ¹³C (100 MHz, DMSO) : 176,0 (OC=O) ; 163,5 (C=O) ; 151,0 (C=O) ; 140,9 (C-6) ; 102,4 (C-5) ; 88,0 (OCH₂O) ; 86,4 (C-1') ; 85,6 (C-4') ; 81,1 (C-2') ; 69,1 (C-3') ; 61,1 (C-5') ; 33,7 (C(O)CH) 18,9 (CH₃, 2C).
10b. (1,56 g, 92%). RMN ¹H (400 MHz, HH-COSY, DMSO): 11,42 (s, 1H, N*H*) ; 7,92 (d, *J* _{H-6/H-5} = 8,1 Hz, 1H, *H*-6) ; 5,94 (d, *J* _{H-1'/H-2'} = 5,5 Hz, 1H, *H*-1 ') ; 5,73 (d, *J* _{H-5/H-6} = 8,1 Hz, 1H, *H*-5) ; 5,42, 5,25 (2d_{AB}, *J*_{AB} = 6,5 Hz 2H, OC*H*₂O) ; 5,35 (s, 1H, O*H*-3') ; 5,22 (s, 1H, O*H*-5'); 4,30 (t, *J* _{H-2'/H-3'} = 5,3 Hz, 1H, *H*-2') ; 4,19 (dd, *J* _{H-3'/H-2'} = 5,2 Hz, *J* _{H-3'/H-4'} = 9,3 Hz, 1H, *H*-3') ; 3,93 (dd, *J* _{H-4'/H-5'b} = 3,1 Hz, *J* = 6,8 Hz, 1H, *H*-4'); 3,70 (d, *J* _{H-5'a/H-5'b} = 12,0 Hz, 1H, *H*-5'a) ; 3,62 (d, *J* _{H-5'b/H-5'a} = 12,0 Hz, 1H, *H*-5'b) ; 2,30 (t, *J* _{CH2α/CH2β}= 7,3 Hz, 2H, C(O)C*H_{2α}*) ; 1,56 (sext, *J* _{CH2β/CH2α}= *J* _{CH2β/CH3γ} = 7,4 Hz, 2H, CH_{2β}), 0,91 (t, *J* _{CH3γ/CH2β} = 7,4 Hz, 3H, CH₃).
   RMN ¹³C (100 MHz, DMSO) : 172,2 (OC=O) ; 163,0 (C=O) ; 151,0 (C=O) ; 140,4 (C-6) ; 101,9 (C-5) ; 87,5 (OCH₂O) ; 86,0 (C-1') ; 85,1 (C-4') ; 80,7 (C-2') ; 68,7 (C-3') ; 60,6 (C-5') ; 35,3 (C(O)CH_{2α}) ; 17,6 (CH2_{β}) ; 13,3 (CH_{3γ}).
10c. (1,25 g, 86%). RMN ¹H (400 MHz, HH-COSY, DMSO): 11,39 (s, ¹H, N*H*) ; 7,93 (d, *J* _{H-6/H-5}= 8,1 Hz, 1H, *H*-6) ; 5,89 (d, *J* _{H-1'/H-2'} = 3,3 Hz, 1H, *H*-1') ; 5,69 (d, *J* _{H-5/H-6} = 8,1 Hz, 1H, *H*-5) ; 5,36, 5,23 (2d_{AB}, *J*_{AB} = 6,5 Hz 2H, OC*H*₂O) ; 4,25 (t, *J* _{H-2'/H-3'} = 5,2 Hz, 1H, *H*-2') ; 4,14 (t, *J* = 4,5 Hz, 1H, *H*-3') ; 3,88 (dd, *J* _{H-4'/H-5'b} = 3,0 Hz, *J* = 7,0 Hz, 1H, *H-*4') ; 3,68-3,56 (m, 2H, H-5'a ; *H*-5'b) ; 2,30 (q, *J* _{CH2α/CH3β}= 7,5 Hz, 2H, C(O)C*H*_{2α}) ; 1,01 (t, *J* _{CH3β/CH2} = 7,5 Hz, 3H, C*H*_{3β})
   NMR ¹³C (100 MHz, DMSO) : 173,0 (OC=O) : 163,0 (C=O) ; 151,5 (C=O) ; 140,4 (C-6) ; 101,9 (C-5) ; 87,7 (OCH₂O) ; 86,1 (C-1') ; 85,0 (C-4') ; 80,8 (C-2') ; 68,6 (C-3') ; 60,5 (C-5') ; 26,8 (C(O)CH_{2α}) ; 8,6 (CH_{3β}).
10d. L'identification de ce composé est identique à celle décrite pour le même composé dans Parey et al, "First Evaluation of Acyloxymethyl or Acylthiomethyl Groups as Biolabile 2'-O-Protections of RNA", Organic Letters, 2006, Vol.8, No.17, 3869-3872.

Ensuite, les composés **11 a** à **11 d** suivants ont été synthétisés :
2'-*O*-*is*obutyryloxyméthyl-5'-*O*-(4,4'-diméthoxytrityl)uridine **11a,** 2'-*O*-butyryloxyméthyl-5'-*O*-(4,4'-diméthoxytrityl)uridine **11b.**
2'-*O*-propionyloxyméthyl-5'-*O*-(4,4'-diméthoxytrityl)uridine **11c**
et 2'-*O*-acétyloxyméthyl-5'-*O*-(4,4'-diméthoxytrityl)uridine **11d.**

Pour cela, les composés **10a** (1,25 g, 3,64 mmol, 1 eq), **10b** (1,46 g, 4,25 mmol, 1, eq), **10c** (1,15 g, 3,49 mmol, 1 eq) et 10d (1,51 g, 4,77 mmol, 1 eq) ont été séchés par trois coévaporations avec de la pyridine anhydre. Puis les solutions de 10a à 10d dans de la pyridine anhydre (20 mL) ont été traitées avec du chlorure de diméthoxytrityle (1,2 eq) ajouté en petites portions en 15 minutes. Les réactions ont été agitées pendant 2 à 4 heures à température ambiante sous argon. A la fin de la réaction, les mélanges ont été concentrés et du CH₂Cl₂a été ajouté. Les solutions ont été versées dans une solution de NaHCO₃ saturée. Des extractions avec du CH₂Cl₂ ont été effectuées et les extraits ont été séchés sur Na₂SO₄. Les mélanges obtenus après élimination du solvant ont été soumis à une chromatographie sur colonne de gel de silice avec un gradient de CH₂Cl₂ (80-100%) dans du cyclohexane avec 1% de pyridine, puis de MeOH (0-1%) dans du CH₂Cl₂ avec 1% de pyridine. Les composés **11a** à **11d** voulus ont été obtenus sous la forme de mousses blanches après évaporation du solvant.
**11a.** (1,89 g, 79%). RMN ¹H (400 MHz, HH-COSY, CDCl₃) 9,40 (s, 1H, N*H*) ; 7,93 (d, *J* _{H-6/H-5} = 8,2 Hz, 1H, *H*-6) ; 7,32-7,08 (m, 10H, Hₐᵣ) ; 6,79-6,75 (m, 3H, Hₐᵣ) ; 5,88 (d, *J* _{H-1'/H-2'} = 1,8 Hz, 1H, *H*-1') ; 5,50, 5,38 (2d_{AB}, *J*_{AB}= 6,3 Hz, 2H, OC*H*₂O) ; 5,23 (d, *J* _{H-5/H-6}= 8,6 Hz, 1H, *H*-5) ; 4,39 (m, 1H, *H*-3') ; 4,25 (dd, *J* _{H-2'/H-1'} = 1,8 Hz, *J* _{H-2'/H-3'} = 5,2 Hz, 1H, *H*-2') ; 3,95 (td, *J* _{H-4'/H-5'a} = *J* _{H-4'/H-5'b} = 2,1 Hz, *J* _{H-4'/H-3'=} 7,6 Hz, 1H, *H*-4') ; 3,72, 3,71 (2s, 6H, OC*H*₃) ; 3,46 (m, 2H, *H*-5'a, *H*-5'b) ; 2,52 (hept, *J* CH/(CH3)2 = 7,0 Hz, 1H, C(O)C*H*) ; 1,11 (2d, *J* _{CH3/CH} = 7,0 Hz, 6H, CH₃)
   RMN ¹³C (100 MHz, CDCl₃) : 176,6 (OC=O) ; 163,4, 150,3 (C=O) ; 158,8-158,7-144,3-135,2-135,0 (Cq arom.) ; 139,7 (C-6) ; 130,2-130,1-129,2-129,0-128,2-128,1-128,0-127,2-125,3-123,8-113,3-113,1 (CH arom.) ; 102,2 (C-5) ; 88,2 (C-1') ; 87,7 (OCH₂O) ; 87,1 (OCq, DMTr) ; 83,2 (C-4') ; 82,1 (C-2') ; 68,5 (C-3') ; 61,2 (C-5') ; 55,3 (OCH₃, DMTr) ; 34,0 (C(O)CH) ; 17,8 (CH₃, iPr).
**11b.** (2,26 g, 79%). RMN ¹H (400 MHz, HH-COSY, CDCl₃) : 9,20 (s, 1H, N*H*) ; 7,91 (d, *J* _{H-6/H-5} = 8,2 Hz, 1H, *H*-6) ; 7,33-7,14 (m, 10H, Hₐᵣ) ; 6,79-6,75 (m, 3H, Hₐᵣ) ; 5,88 (d, *J* _{H-1'/H-2'} = 1,8 Hz, 1H, *H*-1') ; 5,48, 5,38 (2d_{AB}, *J*_{AB} = 6,3 Hz 2H, OC*H*₂O) ; 5,23 (dd, *J* _{H-5/NH} = 1,9 Hz, *J* _{H-5/H-6} = 8,2 Hz, 1H, *H*-5) ; 4,43-4,37 (m, 1H, *H*-3') ; 4,26-4,24 (m, 1H, *H*-2') ; 3,72, 3,71 (2s, 6H, OC*H*₃) ; 3,96-3,94 (m, 1H, *H*-4') ; 3,50-3,42 (m, 2H, *H*-5'a, *H*-5'b) ; 2,47 (d, *J* _{OH-3'/H-3'} = 8,9 Hz, 1H, O*H*-3') ; 2,27 (t, *J* = 7,5 Hz, 2H, C(O)C*H*_{2α}) ; 1.58 (sext, *J* _{CH2β/CH2α} = *J* _{CH2β/CH3γ} = 7.5 Hz, 2H, CH_{2β}); 0,88.(t, *J* _{CH3γ/CH2β} = 7.5 Hz, 3H, C*H*_{3γ}).
   RMN ¹³C (100 MHz, CDCl₃) : 173,1 (OC=O) ; 163,2, 150,2 (C=O) ; 158,8-158,7-144,3-135,2-135,0 (Cq arom.) ; 139,8 (C-6) ; 130,2-130,1-129,1-129,0-128,1-128,0-127,8-127,2-113,3-113,2-113,1 (CH arom.) ; 102,2 (C-5) ; 88,1 (C-1') ; 87,7 (OCH₂O) ; 87,1 (OCq, DMTr) ; 83,2 (C-4') ; 82,0 (C-2') ; 68,6 (C-3') ; 61,2 (C-5') ; 55,3 (OCH₃, DMTr) ; 36,1 (C(O)CH₂) ; 18,1 (CH_{2β}) ; 13.6 (CH_{3γ}).
**11c.** (1,81 g, 82%). RMN ¹H (400 MHz, HH-COSY, CDCl₃) : 9,27 (s, 1H, N*H*) ; 7,91 (d, *J* _{H-6/H-5} = 8,1 Hz, 1H, *H*-6) ; 7,31-7,08 (m, 10H, Hₐᵣ) ; 6,78-6,74 (m, 3H, Hₐᵣ) ; 5,88 (d, *J* _{H-1'/H-2'} = 1,7 Hz, 1H, *H*-1') ; 5,47, 5,39 (2d_{AB}, *J*_{AB} = 6,4 Hz 2H, OC*H*₂O) ; 5,22 (dd, *J* _{H-5/NH} = 1,0 Hz, *J* _{H-5/H-6} = 8,1 Hz, 1H, H-5) ; 4,43-4,37 (m, 1H, H-3') ; 4,25 (m, 1H, *H*-2') ; 3,95 (d, *J* = 7,5 Hz, 1H, H-4') ; 3,72, 3,71 (2s, 6H, OC*H*₃) ; 3,47-3,42 (m, 2H, *H*-5'a, *H*-5'b) ; 2,48 (d, *J* _{OH-3'/H-3'} = 8,5 Hz, 1H, O*H*-3') ; 2,31 (q, *J* = 7.5 Hz, 2H, C(O)CH_{2α}) ; 1,07 (t, *J* _{CH3β/CH2α} = 7,5 Hz, 3H, C*H*_{3β}).
   RMN ¹³C (100 MHz, CDCl₃): 174,0 (OC=O) ; 163,4, 150,2 (C=O) ; 158,7-144,3-141,5-135,3-135,0 (Cq arom.) ; 139,9 (C-6) ; 130,2-130,1-129,1-128,1-128,0-127,8-127,7-127,2-127,1-113,3-113,2 (CH arom.) ; 102,2 (C-5) ; 88,1 (C-1') ; 87,8 (OCH₂O) ; 87,1 (OCq, DMTr) ; 83,2 (C-4') ; 82,0 (C-2') ; 68,6 (C-3') ; 61,2 (C-5') ; 55,3 (2C, OCH₃, DMTr) ; 27,5 (C(O)CH₂) 8,8 (CH_{3β}).
**11d.** L'identification de ce composé est identique à celle décrite pour le même composé dans Parey et al, "First Evaluation of Acyloxymethyl or Acylthiomethyl Groups as Biolabile 2'-O-Protections of RNA, Organic Letters", 2006, Vol.8, No.17, 3869-3872.

Enfin, les monomères de formule III ont été synthétisés.

2'*O*-*is*obutyryloxyméthyl-3'-O-(2-cyanoéthyl *N,N* diisopropylphosphoramidite)-5'-*O*-(4,4'-diméthoxytrityl)uridine **12a**, 2'-*O-*butyryloxyméthyl-3'-*O*-(2-cyanoéthyl-*N,N*-diisopropylphosphoramidite)-5'-*O*-(4,4'-diméthoxytrityl)uridine **12b**, 2'-*O*-propionyloxyméthyl-3'-*O*-(2-cyanoéthyl-*N,N-*diisopropylphosphoramidite)-5'-*O*-(4,4'-diméthoxytrityl)uridine **12c** et 2'-*O-*acétyloxyméthyl-3'-*O*-(2-cyanoéthyl-*N,N*-diisopropylphosphoramidite)-5'-*O*-(4,4'-diméthoxytrityl)uridine **12d.**

Pour cela, les composés **11a** (1,77 g, 2,74 mmol, 1 eq), **11b** (2,17 g, 3,36 mmol, **11c** (1,80 g, 2,85 mmol) et **11d** (1,65 g, 2, 67 mmol) ont été séchés par trois coévaporations avec du CH₃CN anhydre. Puis, le résidu a été dissout dans du CH₂Cl₂ anhydre (14 mL) et un mélange de *N,N-*diisopropyléthylamine (pour **11a :** 859 µL, 4,93 mmol, 1,8 eq; pour **11b :** 1050 µL, 6,06 mmol, 1,8 eq; pour **11c :** 894 µL, 5,13 mmol, 1,8 eq; et pour **11d :** 838 µL, 4,81 mmol, 1,8 eq), et de 2-cyanoéthyl-*N,N*-düsopropylchlorophosphoramidite (pour **11a :** 917 µL, 4,11 mmol, 1,5 eq; pour **11b :** 1120 µL, 5,04 mmol, 1,5 eq; pour **11c** : 954 µL, 4,28 mmol, 1,5 eq; pour **11d :** 895 µL, 4,01 mmol, 1,5 eq) et CH₂Cl₂ (2 mL) a été ajouté goutte à goutte. Le mélange a été agité sous argon, à température ambiante, pendant 3 heures. Après la fin de la réaction, de l'acétate d'éthyle a été ajouté, le mélange réactionnel a été versé dans une solution de NaHCO₃ saturée et des extractions avec de l'AcOEt ont été mises en oeuvre. Le mélange, obtenu après séchage de l'extrait sur Na₂SO₄ et élimination du solvant a été purifié par chromatographie sur colonne de gel de silice avec un gradient de CH₂Cl₂ (50-100%) dans du cyclohexane avec 1% de pyridine. Les phosphoramidites voulus **12a** à **12d** ont été obtenus sous la forme de mousses blanches après évaporation du solvant.
**12a.** (1.63 g, 70%). RMN ³¹P (121 MHz, CD₃CN): δ (ppm): 150.35, 149.15.
**12b.** (1.85 g, 66%). Rmn ³¹P (121 MHz, CD₃CN): δ (ppm): 150.31, 149.09.
**12c.** (1.93 g, 81%). RMN ³¹P (121 MHz, CD₃CN): δ (ppm): 150.30, 149.01.
**12d.** (1.14 g, 52%). RMN ³¹P (121 MHz, CD₃CN): δ (ppm): 150.31, 148.96.

### Exemple 7 : Synthèse d'ARN simple brin de formule I par la chimie des phosphoramidites avec X₃ qui est un groupe isobutyryloxyméthyle ou un groupe butyryloxyméthyle ou un groupe propionyloxyméthyle ou un groupe acétyloxyméthyle.

A partir des monomères obtenus **12a-d** à l'exemple 6, des ARN simple brin ont été synthétisés.

Ces ARN protégés de formule I ont été préparés selon le procédé identique à celui décrit dans l'exemple 5 pour l'obtention des ON4 à ON5. Les ARN notés ON6 à ON9 de séquence identique U₁₉ TT ont été obtenus avec les résultats montrés au tableau 3 suivant :

**Tableau 3**

| ON^{[a]} U₁₉ TT | X₃ | CT^{[b]} | OY^{[c]} | AY^{[d]} | Matériau brut^{[e]} |
|---|---|---|---|---|---|
| 6 | *i*sobutyryloxyméthyle | 180 | 75.9 | 98.5 | 122 |
| 7 | Butyryloxyméthyle | 180 | 68.4 | 98.1 | 130 |
| 8 | Propionyloxyméhtyle | 180 | 77.9 | 98.7 | 100 |
| 9 | Acétyloxyméthyle | 180 | 70.7 | 98.3 | 120 |

| | | | | | |
|---|---|---|---|---|---|
| [a] ON = oligoribonucléotides [b] = temps de couplage (s) dans le cycle de synthèse automatisé [c] OY = rendement global de couplage (%) [d] AY = rendement de couplage moyen par étape [e] = matériau brut global (Unités D.O = densité optique) mesuré à 260 nm par absorption UV | | | | | |

Il est à noter que les groupes X₃ isobutyryloxyméthyle et butyryloxyméthyle ont été enlevés de l'ARN de formule I en 15 min par le traitement à l'ammoniaque concentré et les groupes X₃ propionyloxyméthyle et acétyloxyméthyle ont été enlevés en moins de 5 min par le traitement à l'ammoniaque concentré même si ce traitement a été prolongé jusqu'à 1 h30 pour rompre complètement le lien succinyle avec le support solide et libérer les ARN.

### Exemple 8 : Digestion enzymatique d'ARN

Les ARN ON4 et ON5 obtenus à l'exemple 5 (2 unités de DO à 260 nm) ont été incubés avec une nucléase P1 (0,25 unités) (spécifique des coupures de liaisons internucléosidiques 3'-5') à 37°C pendant 48h.

Puis, une phosphatase alcaline (2,5 unités) et un tampon (50 mM Tris-HCl, pH 9,3, contenant 1 mM de MgCl₂, 0,1 mM de ZnCl₂ et 1 mM de spermidine; concentrations finales) ont été ajoutés pour donner un volume total de 115 µl et le mélange a été incubé à 37°C pendant 24h supplémentaires. Le mélange réactionnel a été analysé par HPLC.

Les ARN ont été complètement dégradés pour donner les quatre ribonucléosides naturels ce qui prouve l'intégrité des liaisons internucléotidiques 3'-5'. Aucune liaison non naturelle 2'-5' n'a été détectée.

### Exemple 9 : Test in vitro d'un siARN obtenu par le procédé de l'invention

L'activité du duplex siARN obtenu à partir des ARN simple brin ON4 et ON5 hybridés ensemble a été évaluée dans un test ARN interférence qui cible l'ARN messager de l'oncogène Ret/PTC1 impliqué dans le cancer papillaire de la thyroïde.

Cette activité du duplex siARN ON4/ON5 obtenu par la méthode de l'invention a été comparée à l'activité du même duplex siARN AS fourni par Eurogentec et synthétisé par une autre voie de synthèse (méthode 2'-TBDMS).

Les cellules utilisées pour tester le siARN obtenu étaient des fibroblastes murins NIH/3T3 transfectés de façon stable par un vecteur pBAB exprimant l'oncogène humain Ret/PTC1. La culture de cellules a été effectuée dans un milieu DMEM (GIBCO) contenant 10% de sérum bovin de nouveau né inactivé par la chaleur (10%, GIBCO), de la pénicilline (100 U/ml), de la streptomycine (100 µg/ml, GIBCO), et de la puromycine (2,5 µg/ml Sigma) à 37°C avec 5% de CO₂ dans une atmosphère humide.

Cette culture de cellules a été ensuite traitée avec le siARN obtenu à partir des ARN simple brin ON4 et ON5 ainsi qu'avec le siARN AS commercial.

Un jour avant le traitement, 3.10⁵ cellules ont été ensemencées sur des plaques à six puits. Une transfection a été effectuée en mélangeant 0,05 nmol de siARN dans 50 µl de tampon Hepes 10 mM, pH 7,2, 100 mM de NaCl avec 2 µg de cytofectine (GTS) dans 50 µl du même tampon. Après 10 minutes d'incubation à température ambiante, les complexes ont été ajoutés aux cellules dans 900 µl de milieu de culture frais contenant du sérum pendant 24h. L'expérience a été effectuée en triple. Les séquences de contrôle du siARN sont SEQ ID NO: 6 : 5'-GCCAGUGUCACCGUCAAGGdAdG-3' et SEQ ID NO: 7 : 5'-CCUUGACGGUGACACUGGCdTdT-3' et ont été fournies par Eurogentec. Ce sont des séquences aléatoires non spécifiques de l'ARNm visé.

Puis, la détection de l'expression de l'ARNm Ret/PTC1 a été effectuée par RT-PCR. L'extraction de l'ARNm a été effectuée avec le réactif TRIzol (Invitrogen) comme indiqué par le fabricant. Après détermination de la concentration par spectrométrie UV, 1 µg de l'ARN total provenant soit des cellules de contrôle, soit des cellules traitées ont été incubés avec Mo-MuLV RT (Promega), comme indiqué par le fabricant, dans 20 µl de volume final pendant 1 h à 42°C. Puis l'expression de l'ARNm Ret/PTC1 a été déterminée par PCR sur 2 µl de produits de transcription inverse dans 50 µl de milieu réactionnel comprenant la Taq Polymerase (Ozyme).

Les amorces suivantes ont été utilisées pour l'amplification de Ret/PTC1 (290 pb) amorce antisens : SEQ ID NO: 8: 5'-CTGCTTCAGGACGTTGAA-3' et amorce sens SEQ ID NO: 9: 5'-AGATAGAGCTGGAGACCTAC-3'.

Le GAPDH (531 pb), gène codant pour l'enzyme glyceraldehyde 3-phosphate deshydrogenase, a été utilisé comme contrôle du fonctionnement de la transcription inverse (RT) avec les séquences amorces SEQ ID NO :10: 5'-GACAACTCACTCAAGATTGTCAG-3' et SEQ ID NO :11: 5'-CATTGTCATACCAGGAAATG-3'.

Les produits de PCR ont été obtenus après 21 ou 32 cycles, respectivement, et analysés par électrophorèse sur gel d'agarose à 2% avec un tampon Tris Acétate EDTA (TAE) [0,5x]. Les fragments d'ADN ont été détectés sous illumination UV après coloration avec du bromure d'éthydium et ensuite une quantification a été effectuée avec un système d'analyse par ordinateur couplé à une caméra (Syngene). Les expériences ont été effectuées en triple.

Ces différentes expériences (faites en triple) ont montré que le siARN double brin obtenu à partir des ARN ON4 et ON5, eux-mêmes obtenus par la méthode de l'invention avait une meilleure activité pour rendre silencieux le gène (60% d'inhibition) que le siARN AS double brin de même séquence acheté et fabriqué par la méthode chimique de protection avec le TBDMS (40% d'inhibition). Cette meilleure activité pourrait être expliquée par une pureté plus élevée de l'ARN double brin de l'invention purifié par RP-HPLC en comparaison au siARN AS purifié par PAGE.

Dans tous les cas, ce résultat confirme l'intégrité et la pureté des ARN synthétisés par la méthode de l'invention.

Il apparaitra clairement à l'homme de l'art que tout autre groupement protecteur des nucléobases, et des hydroxyles en position 3' et 5' du ribose bien connus, peuvent être utilisés, à condition que le groupement protecteur de l'hydroxyle en position 3' soit baso-labile de type acyloxyalkyle ou acylthioalkyle.

De plus, il apparaîtra clairement à l'homme du métier que le traitement avec l'ammoniaque utilisé pour déprotéger l'hydroxyle en position 2' et les fonctions amines exocycliques des nucléobases de l'ARN synthétisé permet également, et en même temps, de libérer l'ARN de son support solide, lorsqu'il est lié au support solide par un lien baso-labile tel qu'un lien succinyle ou Q-linker.

De plus, bien que dans la description qui précède et les revendications annexées, le groupe protecteur X₁ ait été décrit comme étant choisi parmi des groupes acido-labiles, il apparaitra clairement à l'homme de l'art que des groupes fluor-labiles ou baso-labiles appropriés connus de l'homme du métier pourront également être utilisés pour protéger l'hydroxyle en position 5' du ribose.

## Revendications

1. Procédé de libération d'un ARN simple brin à partir d'un ARN simple brin, protégé et lié par un lien à un support solide, de formule I suivante : 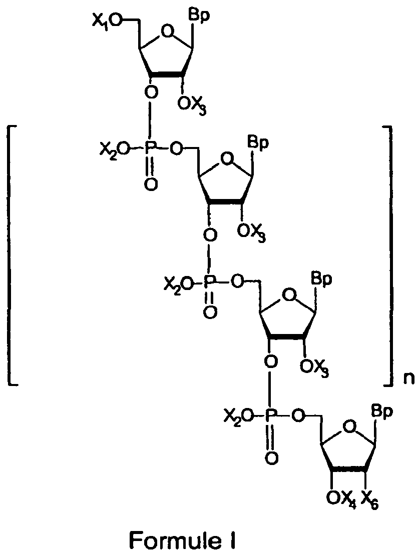 dans laquelle :
- X₁ est H ou un groupe protecteur d'hydroxyle choisi parmi un groupe diméthoxytrityle, un groupe monométhoxytrityle et un groupe pixyle, de préférence un groupe diméthoxytrityle,
- X₂ est H ou un groupe protecteur du phosphate β-éliminable, de préférence un groupe cyanoéthyle,
- X₃ est un groupe baso-labile protecteur des hydroxyles en position 2' du ribose de formule A suivante : 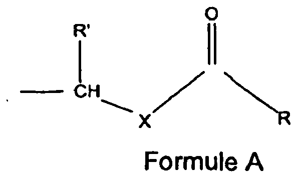
dans laquelle X est O ou S, R' est H ou CH₃, et R est choisi parmi un groupe alkyle en C₁ à C₄ linéaire ou ramifié et un groupe R₁-O-R₂ dans lequel R₁ est un groupe alkyle en C₁ à C₂ et R₂ est un groupe CH₃ ou CH₂CH₂-O-CH₃ ou aryle,
- X₄ représente l'ensemble lien-support,
- X₆ est H ou un groupe OX_{3 ou} OAc,
- Bp est une nucléobase thymine naturelle ou modifiée lorsque X₆ est H ou uracile naturelle ou modifiée lorsque X₆ est OX₃ ou OAc, ou adénine naturelle ou modifiée protégée, ou cytosine naturelle ou modifiée protégée, ou guanine naturelle ou modifiée protégée quelque soit X₆, et
- n est un entier supérieur ou égal à 0,
**caractérisé en ce qu'**il comprend une étape a) de traitement de l'ARN simple brin protégé lié à un support de formule 1 avec une base choisie parmi la pipéridine, la 1,8-diazabicyclo[5.4.0]undec-7-ène (DBU), la triéthylamine, à température ambiante, pour libérer le phosphate des liens internucléosidiques 3'-5', lorsque X₂ est différent de H, suivie d'une étape b) de traitement de l'ARN partiellement libéré obtenu à l'étape a), avec une base choisie parmi l'ammoniaque concentré, la méthylamine, le carbonate de potassium, à température ambiante.

2. Procédé selon la revendication 1 **caractérisé en ce que**, dans la formule I, X₃ est un groupe pivaloyloxyméthyle, ou un groupe isobutyryloxyméthyle, ou un groupe butyryloxyméthyle ou un groupe propionyloxyméthyle, ou un groupe acétyloxyméthyle.

3. Procédé selon l'une quelconque des revendications précédentes **caractérisé en ce que**, dans la formule I, la nucléobase Bp est l'uracile naturelle ou modifiée.

4. Procédé selon la revendication 1 ou 2 **caractérisé en ce que**, dans la formule I, les quatre nucléobases uracile, adénine, cytosine et guanine, naturelles ou modifiées, sont présentes et **en ce qu'**à l'étape b) l'enlèvement du groupe protecteur X₃ est effectuée par traitement avec de l'ammoniaque, puis ajout de 15% en volume par rapport au volume total d'ammoniaque, d'isopropylamine, puis évaporation sous pression réduite du milieu de déprotection.

5. Procédé selon l'une quelconque des revendications précédentes **caractérisé en ce que** l'ARN, protégé et lié à un support, de formule I, est lié à un support solide par un lien baso-labile.

6. Procédé de synthèse d'un ARN simple brin **caractérisé en ce qu'**il comprend les étapes suivantes :
a) liaison à un support solide d'un monomère de formule II suivante 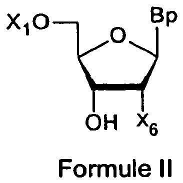 dans laquelle :
- X₁ est un groupe diméthoxytrityle,
- X₆ est H ou un groupe OAc ou OX₃ dans lequel X₃ est un groupe de formule A suivante : 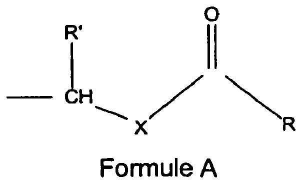
dans laquelle X est O ou S, R' est H ou CH₃, et R est choisi parmi un groupe alkyle en C₁ à C₄ linéaire ou ramifié et un groupe R₁-O-R₂ dans lequel R₁ est un groupe alkyle en C₁ à C₂ et R₂ est un groupe CH₃ ou CH₂CH₂-O-CH₃ ou aryle,
- Bp est une nucléobase thymine naturelle ou modifiée lorsque X₆ est H ou une nucléobase uracile naturelle ou modifiée lorsque X₆ est OAc ou OX₃ ou alors une nucléobase adénine naturelle ou modifiée protégée ou une nucléobase cytosine naturelle ou modifiée protégée ou une nucléobase guanine naturelle ou modifiée protégée quelque que soit X₆,
b) assemblage, avec le monomère de formule II lié à son support obtenu à l'étape a), d'au moins un monomère de formule III suivante : 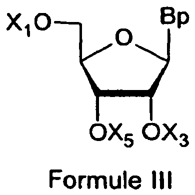 dans laquelle X₁, Bp, X₃ sont tels que définis pour la formule II et X₅ est un groupe 2-cyanoéthyl-*N,N*-diisopropylphosphoramidite, ce par quoi on obtient un ARN simple brin protégé lié à un support de formule I, et
c) optionnéllement traitement par un milieu acide de l'assemblage obtenu à l'étape b), et
d) libération de l'ARN simple brin protégé lié à un support obtenu à l'étape b) ou à l'étape c), par le procédé de libération selon l'une quelconque des revendications 1 à 6.

7. Procédé de synthèse d'un ARN simple brin selon la revendication 6 **caractérisé en ce qu'**il comprend de plus, avant l'étape b), une étape a') de synthèse d'un monomère de formule III consistant en les étapes suivantes :
a) protection des amines exocycliques des nucléobases Bp, lorsque la nucléobase Bp est différente de l'uracile naturelle ou modifiée,
b) protection de l'hydroxyle en position 5' du sucre ribose,
c) protection de l'hydroxyle en position 2' du sucre ribose avec un groupe de formule A, et
d) fonctionnalisation de l'hydroxyle en position 3' du sucre ribose avec un groupe 2-cyanoéthyl -N,N-diisopropylphosphoramidite.

8. Procédé de synthèse d'un ARN double brin **caractérisé en ce qu'**il comprend la synthèse d'un ARN simple brin selon le procédé de la revendication 6 et l'hybridation de l'ARN simple brin ainsi synthétisé avec un ARN simple brin ayant une séquence complémentaire.

9. Procédé de synthèse selon la revendication 7 ou 8 **caractérisé en ce que** l'ARN double brin est un siARN.

10. Procédé de synthèse d'un monomère de formule III suivante: 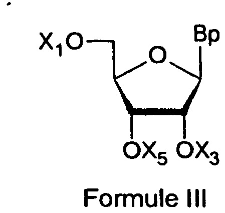 dans laquelle
- Bp est une nucléobase uracile naturelle ou modifiée ou une nucléobase adénine naturelle ou modifiée protégée ou une nucléobase cytosine naturelle ou modifiée protégée ou une nucléobase guanine naturelle ou modifiée protégée,
- X₁ est un groupe diméthoxytrityle,
- X₃ est un groupe baso-labile de formule A suivante : 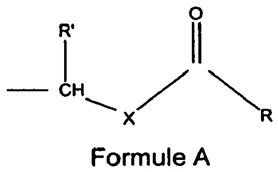
dans laquelle X est O ou S, R' est H ou CH₃, et R est choisi parmi un groupe alkyle en C₁ à C₄ linéaire ou ramifié et un groupe R₁-O-R₂ dans lequel R₁ est un groupe alkyle en C₁ à C₂ et R₂ est un groupe CH₃ ou CH₂CH₂-O-CH₃ ou aryle,
- X₅ est un groupe 2-cyanoéthyl-N,N-dilsopropylphosphoramidite à partir d'un monomère ribonucléoside de formule IV suivante : 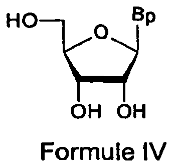
dans laquelle Bp est une nucléobase naturelle ou modifiée uracile, adénine, cytosine, ou guanine,
**caractérisé en ce qu'**il consiste en les étapes suivantes :
a) protection des amines exocycliques des nucléobases Bp, lorsque la nucléobase Bp est différente de l'uracile naturelle ou modifiée,
b) protection de l'hydroxyle en position 5' du sucre ribose,
c) protection de l'hydroxyle en position 2' du sucre ribose avec un groupe protecteur de formule A, et
d) fonctionnalisation de l'hydroxyle en position 3' du sucre ribose avec un groupe 2-cyanoéthyl -*N,N*-diisopropylphosphoramidite.

11. Procédé selon la revendication 10 **caractérisé en ce qu'**à l'étape c) le groupe protecteur est un groupe de formule A choisi parmi un groupe pivaloyloxyméthyle, un groupe isobutyryloxyméthyle, un groupe butyryloxyméthyle, un groupe propionyloxyméthyle, et un groupe acétyloxyméthyle.

12. Procédé selon la revendication 10 ou 11 **caractérisé en ce qu'**à l'étape c), le groupe de formule A est un groupe pivaloyloxyméthyle.

13. Procédé selon l'une quelconque des revendications 10 à 12 **caractérisé en ce qu'**à l'étape b), le groupe protecteur est un groupe diméthoxytrityle

14. Procédé selon l'une quelconque des revendications 10 à 13 **caractérisé en ce que** la nucléobase est la cytosine naturelle ou modifiée et **en ce que** le groupe protecteur à l'étape a) est un groupe acétyle.

15. Procédé selon l'une quelconque des revendications 10 à 13 **caractérisé en ce que** la nucléobase est l'adénine naturelle ou modifiée et **en ce que** le groupe protecteur à l'étape a) est un groupe phénoxyacétyle.

16. Procédé selon l'une quelconque des revendications 10 à 13 **caractérisé en ce que** la nucléobase est la guanine naturelle ou modifiée et **en ce que** le groupe protecteur à l'étape a) est un groupe *tert*-butylphénoxyacétyle ou iso-propylphénoxyacétyle.

## Claims

1. A process for the release of a single strand RNA from a single strand RNA protected and bound by a linker to a solid support, of formula I below: 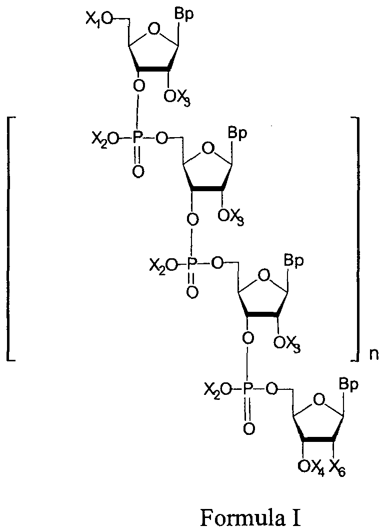 in which:
- X₁ is H or a hydroxyl protecting group selected from a dimethoxytrityl, a monomethoxytrityl and a pixyl group, preferably a dimethoxytrityl group,
- X₂ is H or a group protecting β-removably phosphate, preferably a cyanoethyl group,
- X₃ is a base-labile group protecting hydroxyls in the 2' position of the ribose of formula A below: 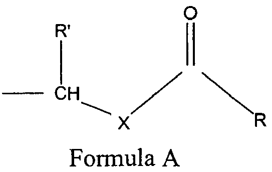
in which X is O or S, R' is H or CH₃, and R is selected from a straight or branched C₁ to C₄ alkyl group and a R₁-O-R₂ group in which R₁ is a C₁ to C₂ alkyl group and R₂ is a CH₃ or CH₂CH₂-O-CH₃ or aryl group,
- X₄ represents the linker-substrate unit,
- X₆ is H or an OX₃ or OAc group,
- Bp is a natural or modified thymine nucleotide base, when X₆ is H or a natural or modified uracil base when X6 is OX₃ or OAc, or protected natural or modified adenine, or protected natural or modified cytosine, or protected natural or modified guanine regardless of the nature of X₆, and
- n is an integer number greater than or equal to 0,
**characterised in that** it comprises a step a) of treatment of the protected single strand RNA bound to a support of formula I with a base selected from piperidine, 1,8-diazabicyclo[5.4.0]undec-7-ene (DBU), or triethylamine at ambient temperature to release the phosphate in the 3'-5' internucleoside linkers when X₂ is not H, followed by a step b) of treatment of the partly-released RNA obtained in step a) with a base selected from concentrated ammonia, methylamine or potassium carbonate, at ambient temperature.

2. The process according to claim 1, **characterised in that** in formula I X₃ is a pivaloxymethyl group, or an isobutyryloxymethyl group, or a butyryloxymethyl group or a propionyloxymethyl group, or an acetyloxymethyl group.

3. The process according to any one of the preceding claims, **characterised in that** in formula I the nucleotide base Bp is natural or modified uracil.

4. The process according to claim 1 or 2, **characterised in that** in formula I the four nucleotide bases natural or modified uracil, adenine, cytosine and guanine are present and **in that** in step b) the X₃ protecting group is removed by treatment with ammonia, after which 15% by volume of isopropylamine based on the total volume of ammonia is added, followed by evaporation of the deprotection medium under reduced pressure.

5. The process according to any one of the preceding claims, **characterised in that** the RNA which is protected and bound to a support of formula I is bound to a solid support through a base-labile linker.

6. The process for the synthesis of a single strand RNA **characterised in that** it comprises the following steps:
a) binding of a monomer of formula II below to a solid support 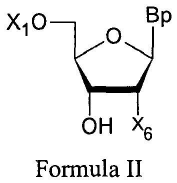 in which:
- X₁ is a dimethoxytrityl group,
- X₆ is H or an OAc or OX₃ group in which X₃ is a group having the following formula A: 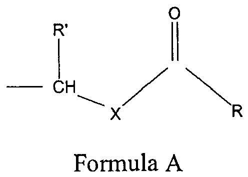
in which X is O or S, R' is H or CH₃, and R is selected from a straight or branched C₁ to C₄ alkyl group and a R₁-O-R₂ group in which R₁ is a C₁ to C₂ alkyl group and R₂ is a CH₃ or CH₂CH₂-O-CH₃ or aryl group,
- Bp is a natural or modified thymine nucleotide base when X₆ is H or a natural or modified uracil base when X₆ is OAc or OX₃ or a protected natural or modified adenine nucleotide base, or a protected natural or modified cytosine nucleotide base, or a protected natural or modified guanine nucleotide base regardless of the nature of X₆,
b) assembly of at least one monomer of formula III below with the monomer of formula II bound to its support obtained in step a): 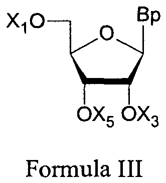 in which X₁, Bp, X₃ are as defined for formula II and X₅ is a 2-cyanoethyl-N,N-diisopropylphosphoramidite group, through which a protected single strand RNA bonded to a support of formula I is obtained, and
c) optionally treating with an acid medium the assembly obtained in step b), and
d) release of the protected single strand RNA bonded to a support obtained in step b) or step c) by the release process according to any one of claims 1 to 6.

7. The process for the synthesis of a single strand RNA according to claim 6, **characterised in that** it further comprises, prior to step b), a step a') of synthesis of a monomer of formula III comprising the following steps:
a) protection of the exocyclic amines of nucleotide bases Bp when nucleotide base Bp is not natural or modified uracil,
b) protection of the hydroxyl in the 5' position of the ribose sugar,
c) protection of the hydroxyl in the 2' position of the ribose sugar with a group of formula A, and
d) functionalising the hydroxyl in the 3' position of the ribose sugar with a 2-cyanoethyl-N,N-diisopropylphosphoramidite group.

8. The process for the synthesis of a double strand RNA, **characterised in that** it comprises the synthesis of a single strand RNA according to the process in claim 6 and hybridisation of the so synthesised single strand RNA with a single strand RNA having a complementary sequence.

9. The process of synthesis according to claim 7 or 8, **characterised in that** the double strand RNA is a siRNA.

10. The process of the synthesis of a monomer of formula III below: 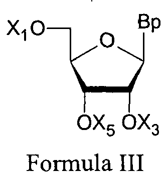 in which
- Bp is a natural or modified uracil nucleotide base or a protected natural or modified adenine nucleotide base or a protected natural or modified cytosine nucleotide base or a protected natural or modified guanine nucleotide base,
- X₁ is a dimethoxytrityl group,
- X₃ is a base-labile group of formula A below: 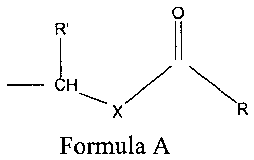
in which X is O or S, R' is H or CH₃, and R is selected from a straight or branched C₁ to C₄ alkyl group and a Ri -O-R₂ group in which R₁ is a C₁ to C₂ alkyl group and R₂ is a CH₃ or CH₂CH₂-O-CH₃ or aryl group,
- X₅ is a 2-cyanoethyl-N,N-diisopropylphosphoramidite group from a ribonucleoside monomer of formula IV below: 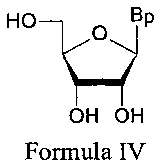
in which Bp is a natural or modified uracil, adenine, cytosine or guanine nucleotide base,
**characterised in that** it comprises the following steps:
a) protection of the exocyclic amines of the Bp nucleotide base when nucleotide base Bp is not natural or modified uracil,
b) protection of the hydroxyl in the 5' position of the ribose sugar,
c) protection of the hydroxyl in the 2' position of the ribose sugar with a protecting group of formula A, and
d) functionalisation the hydroxyl in the 3' position of the ribose sugar with a 2-cyanoethyl-*N,N* diisopropylphosphoramidite group.

11. The process according to claim 10, **characterised in that** in step c) the protecting group is a group of formula A selected from a pivaloyloxymethyl group, an isobutyryloxymethyl group, a butyryloxymethyl group, a propionyloxymethyl group and an acetyloxymethyl group.

12. The process according to claim 10 or 11, **characterised in that** in step c) the group of formula A is a pivaloyloxymethyl group.

13. The process according to any one of claims 10 to 12, **characterised in that** in step b) the protecting group is a dimethoxytrityl group.

14. The process according to any one of claims 10 to 13, **characterised in that** the nucleotide base is natural or modified cytosine and **in that** the protecting group in step a) is an acetyl group.

15. The process according to any one of claims 10 to 13, **characterised in that** the nucleotide base is natural or modified adenine and **in that** the protecting group in step a) is an phenoxyacetyl group.

16. The process according to any one of claims 10 to 13, **characterised in that** the nucleotide base is natural or modified guanine and **in that** the protecting group in step a) is a t-butylphenoxyacetyl or iso-propylphenoxyacetyl group.

## Patentansprüche

1. Verfahren zur Freisetzung einer Einzelstrang-RNA ausgehend von einer geschützten und durch einen Linker an einen festen Träger gebundenen Einzelstrang-RNA der folgenden Formel I: 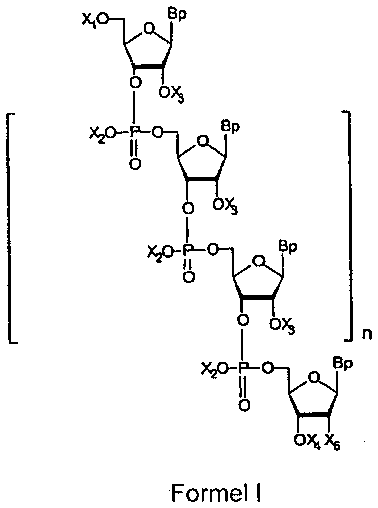 worin
- X₁ H oder eine Hydroxyschutzgruppe ist, die ausgewählt ist aus einer Dimethoxytritylgruppe, einer Monomethoxytritylgruppe, und einer Pixylgruppe, vorzugsweise einer Dimethoxytritylgruppe,
- X₂ H oder eine Schutzgruppe für das β-eliminierbaren Phosphat ist, vorzugsweise eine Cyanethylgruppe,
- X₃ eine basenlabile Schutzgruppe für die Hydroxygruppen in 2'-Position der Ribose der folgenden Formel A ist: 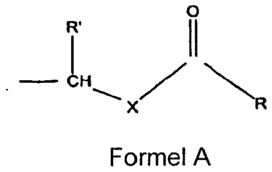 worin X O oder S ist, R' H oder CH₃ ist, und R ausgewählt ist aus einer linearen oder verzweigten C₁-C₄-Alkylgruppe und einer R₁-O-R₂-Gruppe, worin R₁ eine C₁-C₂-Alkylgruppe ist und R₂ eine CH₃- oder CH₂CH₂-O-CH₃-Gruppe oder eine Arylgruppe ist,
- X₄ die Linker-Substrat-Einheit darstellt,
- X₆ H oder eine OX₃- oder OAc-Gruppe ist,
- Bp eine natürliche oder modifizierte Thymin-Nukleobase ist, wenn X₆ H ist, oder natürliches oder modifiziertes Uracil, wenn X₆ OX₃ oder OAc ist, oder ungeachtet von X₆ geschütztes natürliches oder modifiziertes Adenin oder geschütztes natürliches oder modifiziertes Cytosin oder geschütztes natürliches oder modifiziertes Guanin ist, und
- n eine ganze Zahl größer oder gleich 0 ist,
**dadurch gekennzeichnet, dass** es umfasst: einen Schritt a) der Behandlung der an einen Träger gebundenen geschützten Einzelstrang-RNA der Formel I mit einer Base, ausgewählt aus Piperidin, 1,8-Diazabicyclo[5.4.0]undec-7-en (DBU) und Triethylamin, bei Raumtemperatur zur Freisetzung des Phosphats der 3'-5'-Internukleosidlinker, wenn X₂ von H verschieden ist, gefolgt von einem Schritt b) der Behandlung der in Schritt a) erhaltenen teilweise freigesetzten RNA mit einer Base, ausgewählt aus konzentriertem Ammoniak, Methylamin und Kaliumcarbonat, bei Raumtemperatur.

2. Verfahren nach Anspruch 1, **dadurch gekennzeichnet, dass** X₃ in Formel I eine Pivaloyloxymethylgruppe oder eine Isobutyryloxymethylgruppe oder eine Butyryloxymethylgruppe oder eine Propionyloxymethylgruppe oder eine Acetyloxymethylgruppe ist.

3. Verfahren nach einem der vorhergehenden Ansprüche, **dadurch gekennzeichnet, dass** die Nukleobase Bp natürliches oder modifiziertes Uracil ist.

4. Verfahren nach Anspruch 1 oder 2, **dadurch gekennzeichnet, dass** in Formel I die vier Nukleobasen Uracil, Adenin Cytosin und Guanin, natürlich oder modifiziert, vorliegen, und dadurch, dass in Schritt b) die Abspaltung der Schutzgruppe X₃ durch Behandlung mit Ammoniak, anschließende Zugabe von, bezogen auf das Gesamtvolumen von Ammoniak, 15 Vol-% Isopropylamin und dann Eindampfen des Entschützungsmediums unter reduziertem Druck erfolgt.

5. Verfahren nach einem der vorhergehenden Ansprüche, **dadurch gekennzeichnet, dass** die geschützte und an einen Träger gebundene RNA der Formel I durch einen basenlabilen Linker an einen festen Träger gebunden ist.

6. Verfahren zur Synthese einer Einzelstrang-RNA, **dadurch gekennzeichnet, dass** es die folgenden Schritte umfasst:
a) Binden eines Monomers der folgenden Formel II an einen festen Träger 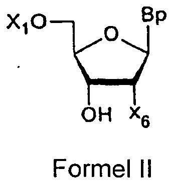 worin
- X₁ eine Dimethoxytritylgruppe ist,
- X₆ H oder eine OAc- oder OX₃-Gruppe ist, worin X₃ eine Gruppe der folgenden Formel A ist: 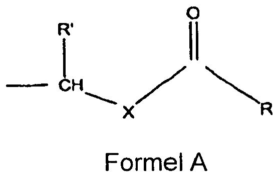 worin X O oder S ist, R' H oder CH₃ ist, und R ausgewählt ist aus einer linearen oder verzweigten C₁-C₄-Alkylgruppe und einer R₁-O-R₂-Gruppe, worin R₁ eine C₁-C₂-Alkylgruppe ist und R₂ eine CH₃- oder CH₂CH₂-O-CH₃-Gruppe oder eine Arylgruppe ist,
- Bp eine natürliche oder modifizierte Thymin-Nukleobase ist, wenn X₆ H ist, oder eine natürliche oder modifizierte Uracil-Nukleobase, wenn X₆ OAc oder OX₃ ist, oder ungeachtet X₆ eine geschützte natürliche oder modifizierte Adenin-Nukleobase oder eine geschützte natürliche oder modifizierte Cytosin-Nukleobase oder eine geschützte natürliche oder modifizierte Guanin-Nukleobase ist,
b) Zusammenbauen, mit dem in Schritt a) erhaltenen, an seinen Träger gebundenen Monomer der Formel II, wenigstens eines Monomers der folgenden Formel III: 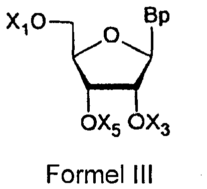 worin X₁, Bp und X₃ wie für Formel II definiert sind, und X₅ eine 2-Cyanethyl-N,N-diisopropylphosphoramidit-Gruppe ist, wodurch eine an einen Träger gebundene geschützte Einzelstrang-RNA der Formel I erhalten wird, und
c) gegebenenfalls Behandlung der in Schritt b) erhaltenen Einheit mit einem sauren Medium, und
d) Freisetzen der in Schritt b) oder in Schritt c) erhaltenen, an einen Träger gebundenen geschützten Einzelstrang-RNA mit dem Verfahren zur Freisetzung nach einem der Ansprüche 1 bis 5.

7. Verfahren zur Synthese einer Einzelstrang-RNA nach Anspruch 6, **dadurch gekennzeichnet, dass** es weiterhin vor Schritt b) einen Schritt a') der Synthese eines Monomers der Formel III bestehend aus den folgenden Schritten umfasst:
a) Schützen der exocyclischen Amine der Nukleobasen Bp, wenn die Nukleobase Bp kein natürliches oder modifiziertes Uracil ist,
b) Schützen des Hydroxyls in 5'-Position des Ribosezuckers,
c) Schützen des Hydroxyls in 2'-Position des Ribosezuckers mit einer Gruppe der Formel A, und
d) Funktionalisieren des Hydroxyls in 3'-Position des Ribosezuckers mit einer 2-Cyanethyl-N,N-diisopropylphosphoramidit-Gruppe.

8. Verfahren zur Synthese einer Doppelstrang-RNA, **dadurch gekennzeichnet, dass** es die Synthese einer Einzelstrang-RNA nach dem Verfahren von Anspruch 6 und die Hybridisierung der so synthetisierten Einzelstrang-RNA mit einer Einzelstrang-RNA mit einer komplementären Sequenz umfasst.

9. Syntheseverfahren nach Anspruch 7 oder 8, **dadurch gekennzeichnet, dass** die Doppelstrang-RNA eine siRNA ist.

10. Verfahren zur Synthese eines Monomers der folgenden Formel III: 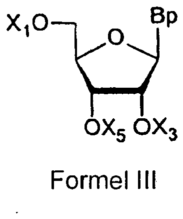 worin
- Bp eine natürliche oder modifizierte Uracil-Nukleobase oder eine geschützte natürliche oder modifizierte Adenin-Nukleobase oder eine geschützte natürliche oder modifizierte Cytosin-Nukleobase oder eine geschützte natürliche oder modifizierte Guanin-Nukleobase ist,
- X₁ eine Dimethoxytritylgruppe ist,
- X₃ eine basenlabile Gruppe der folgenden Formel A ist: 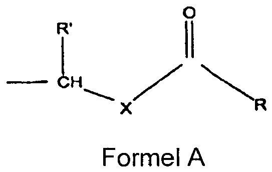 worin X O oder S ist, R' H oder CH₃ ist, und R ausgewählt ist aus einer linearen oder verzweigten C₁-C₄-Alkylgruppe und einer R₁-O-R₂-Gruppe, worin R₁ eine C₁-C₂-Alkylgruppe ist und R₂ eine CH₃- oder CH₂CH₂-O-CH₃-Gruppe oder eine Arylgruppe ist,
- X₅ eine 2-Cyanethyl-*N,N*-diisopropylphosphoramidit-Gruppe ist, ausgehend von einem Ribonukleosidmonomer der folgenden Formel IV 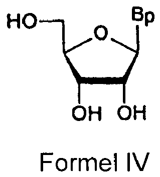
worin Bp eine natürliche oder modifizierte Uracil-, Adenin-, Cytosin- oder Guanin-Nukleobase ist,
**dadurch gekennzeichnet, dass** es aus den folgenden Schritten besteht:
a) Schützen der exocyclischen Amine der Nukleobasen Bp, wenn die Nukleobase Bp kein natürliches oder modifiziertes Uracil ist,
b) Schützen des Hydroxyls in 5'-Position des Ribosezuckers,
c) Schützen des Hydroxyls in 2'-Position des Ribosezuckers mit einer Schutzgruppe der Formel A, und
d) Funktionalisieren des Hydroxyls in 3'-Position des Ribosezuckers mit einer 2-Cyanethyl-N,N-diisopropylphosphoramidit-Gruppe.

11. Verfahren nach Anspruch 10, **dadurch gekennzeichnet, dass** die Schutzgruppe in Schritt c) eine Gruppe der Formel A ist, die ausgewählt ist aus einer Pivaloyloxymethylgruppe, einer Isobutyryloxymethylgruppe, einer Butyryloxymethylgruppe, einer Propionyloxymethylgruppe und einer Acetyloxymethylgruppe.

12. Verfahren nach Anspruch 10 oder 11, **dadurch gekennzeichnet, dass** die Gruppe der Formel A in Schritt c) eine Pivaloyloxymethylgruppe ist.

13. Verfahren nach einem der Ansprüche 10 bis 12, **dadurch gekennzeichnet, dass** die Schutzgruppe in Schritt b) eine Dimethoxytritylgruppe ist.

14. Verfahren nach einem der Ansprüche 10 bis 13, **dadurch gekennzeichnet, dass** die Nukleobase natürliches oder modifiziertes Cytosin ist und dass die Schutzgruppe in Schritt a) eine Acetylgruppe ist.

15. Verfahren nach einem der Ansprüche 10 bis 13, **dadurch gekennzeichnet, dass** die Nukleobase natürliches oder modifiziertes Adenin ist und dass die Schutzgruppe in Schritt a) eine Phenoxyacetylgruppe ist.

16. Verfahren nach einem der Ansprüche 10 bis 13, **dadurch gekennzeichnet, dass** die Nukleobase natürliches oder modifiziertes Guanin ist und dass die Schutzgruppe in Schritt a) eine *tert*-Butylphenoxyacetyl- oder Isopropylphenoxyacetylgruppe ist.
